(19) Europäisches Patentamt
European Patent Office
Office européen des brevets

(11) Veröffentlichungsnummer: **0 041 047**
**A2**

(12) **EUROPÄISCHE PATENTANMELDUNG**

(21) Anmeldenummer: **81810188.3**

(22) Anmeldetag: **18.05.81**

(51) Int. Cl.³: **C 07 D 499/00**, A 61 K 31/43

(30) Priorität: **22.05.80 CH 4017/80**

(43) Veröffentlichungstag der Anmeldung: **02.12.81 Patentblatt 81/48**

(84) Benannte Vertragsstaaten: **AT BE CH DE FR IT LI LU NL SE**

(71) Anmelder: **CIBA-GEIGY AG, Patentabteilung Postfach, CH-4002 Basel (CH)**

(72) Erfinder: **Schneider, Peter, Dr., Rheinfelderstrasse 21 A/1,, CH-4058 Basel (CH)**
Erfinder: **Scartazzini, Riccardo, Dr., Conrad Ferdinand Meyer-Strasse 38,, CH-4059 Basel (CH)**

(54) **Dioxidverbindungen, Verfahren zu ihrer Herstellung und ihre Verwendung.**

(57) 2,2-Dimethyl-penam-3-carbonsäure-1,1-dioxid-verbindungen der Formel

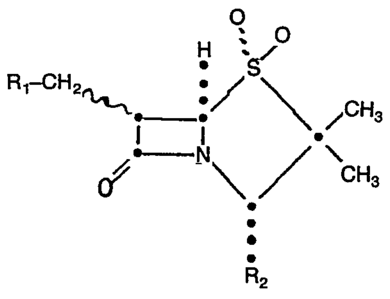

(I)

worin $R_1$ Hydroxy oder veräthertes oder verestertes Hydroxy darstellt und $R_2$ Carboxyl oder geschütztes Carboxyl bedeutet, und Salze von solchen Verbindungen, die eine salzbildende Gruppe aufweisen, haben β-Lactamase-inhibierende Eigenschaften. Pharmazeutische Präparate enthaltend diese Verbindungen, gegebenenfalls in Kombination mit β-Lactamantibiotika, sind besonders zur Bekämpfung von Infektionen geeignet, die durch β-Lactamase-produzierende Mikroorganismen hervorgerufen werden.

Verfahren zur Herstellung von Verbindungen der Formel (I) und Zwischenprodukte.

EP 0 041 047 A2

0041047

4-12867/+

# Dioxidverbindungen, Verfahren zur ihrer Herstellung und ihre Verwendung

Die Erfindung betrifft neue 2,2-Dimethylpenam-3-carbonsäure-1,1-dioxid-verbindungen, Verfahren zur Herstellung dieser Verbindungen, ihre Verwendung zur Inhibierung von β-Lactamasen, pharmazeutische Präparate, die solche Verbindungen gegebenenfalls zusammen mit einer anderen antibiotisch wirksamen β-Lactamverbindung enthalten und die Verwendung von solchen Präparaten.

Es sind bereits einige 1,1-Dioxide von Penam-3-carbonsäureverbindungen mit β-Lactamase-inhibierenden Eigenschaften bekannt geworden. So sind das 2,2-Dimethylpenam-3-carbonsäure-1,1-dioxid und Ester davon aus der belgischen Patentschrift BE 867 859 bekannt. Die 1,1-Dioxide der in 2-Stellung gegebenenfalls durch eine Methylgruppe substituierten Penam-3-carbonsäure und ihrer Ester sind in der europäischen Patentanmeldung 8917 beschrieben. Entsprechende in 6-Stellung durch eine Aminogruppe substituierte Penamverbindungen sind aus der europäischen Patentanmeldung 2927 und solche, die in 6-Stellung durch substituierte Kohlenwasserstoffreste mit mehr als einem Kohlenstoffatom substituiert sind, aus der europäischen Patentanmeldung 5889 bekannt.

In 6-Stellung durch substituiertes Methyl substituierte 2,2-Dimethylpenam-3-carbonsäure-1,1-dioxid-verbindungen sind bisher nicht bekannt geworden. Es wurde die überraschende Feststellung gemacht, dass solche Verbindungen neben antibiotischen insbesondere überlegene β-Lactamase-inhibierende Eigenschaften besitzen. Sie sind daher insbesondere in Kombination mit β-Lactamase-sensiblen

β-Lactamantibiotika, zur Bekämpfung von Infektionen, insbesondere solchen die durch β-Lactamase produzierende Mikroorganismen hervorgerufen werden, geeignet.

Die Erfindung betrifft insbesondere 2,2-Dimethyl-penam-3-carbonsäure-1,1-dioxid-verbindungen der Formel

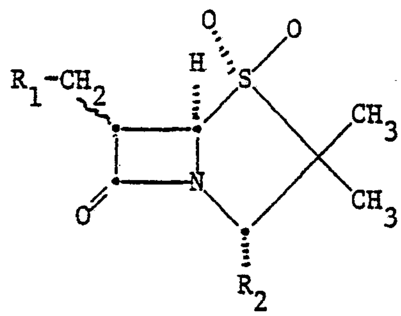

(I),

worin $R_1$ Hydroxy oder veräthertes oder verestertes Hydroxy darstellt und $R_2$ Carboxyl oder geschütztes Carboxyl bedeutet, und Salze von solchen Verbindungen, die eine salzbildende Gruppe aufweisen, Verfahren zur Herstellung dieser Verbindungen, ihre Verwendung zur Inhibierung von β-Lactamasen, pharmazeutische Präparate, die solche Verbindungen gegebenenfalls zusammen mit einer antibiotisch wirksamen β-Lactamverbindung enthalten und die Verwendung von solchen Präparaten.

In der vorliegenden Beschreibung der Erfindung bedeutet der im Zusammenhang mit Definitionen von Gruppen und Verbindungen verwendete Ausdruck "nieder", z.B. in Gruppen wie Niederalkyl, Niederalkylen, Niederalkoxy oder Niederalkanoyl oder in Verbindungen wie niederer Alkohol und dergleichen, dass die entsprechenden Gruppen bzw. Verbindungen, sofern nicht ausdrücklich anders definiert, bis zu 6, bevorzugt bis zu 4 C-Atome enthalten.

Eine verätherte Hydroxygruppe $R_1$ ist eine Gruppe $R_1^a$-O-, worin $R_1^a$ insbesondere einen gegebenenfalls substituierten Kohlenwasserstoffrest oder eine organische Silyl- oder Stannylgruppe darstellt.

Ein gegebenenfalls substituierter Kohlenwasserstoffrest $R_1^a$ hat bis zu 18 C-Atome und ist beispielsweise ein niederaliphatischer, cycloaliphatischer, cycloaliphatisch-aliphatischer, aromatischer oder araliphatischer Rest, der beispielsweise durch Niederalkyl, Niederalkoxy, Halogen, Oxo, Hydroxy, gegebenenfalls funktionell abgewandeltes Carboxy, gegebenenfalls niederalkyliertes Amino oder eine entsprechende geschützte Gruppe, oder auch durch Heterocyclyl substituiert sein kann.

Ein niederaliphatischer Rest $R_1^a$ ist beispielsweise Niederalkyl, insbesondere mit 1-6, bevorzugt 1-4, C-Atomen, z.B. Methyl, Aethyl, Propyl oder Butyl, Niederalkenyl, insbesondere mit 2-5 C-Atomen, z.B. Vinyl, Propenyl oder Butenyl, oder Niederalkinyl, insbesondere mit 2-5 C-Atomen, z.B. Aethinyl oder Propinyl.

Ein cycloaliphatischer Rest $R_1^a$ ist Cycloalkyl mit 3-8, insbesondere 3-6 C-Atomen, z.B. Cyclopropyl, Cyclobutyl, Cyclopentyl oder Cyclohexyl.

Ein cycloaliphatisch-aliphatischer Rest $R_1^a$ ist einer der genannten aliphatischen Reste, der durch einen der genannten cycloaliphatischen Reste substituiert ist, z.B. Cyclopropylmethyl, oder -äthyl, oder Cyclohexylmethyl oder -äthyl.

Ein aromatischer Rest $R_1^a$ ist insbesondere Phenyl oder Naphthyl.

Ein araliphatischer Rest $R_1^a$ ist einer der genannten aliphatischen Reste, der durch einen bis drei der genannten aromatischen Reste substituiert ist, beispielsweise Mono-, Di- oder Triphenylniederalkyl, z.B. Benzyl, Phenäthyl, Diphenylmethyl oder Trityl.

Von den substituierten Kohlenwasserstoffresten $R_1^a$ seien genannt Niederalkoxyniederalkyl, z.B. Methoxymethyl, 1- oder 2-Methoxyäthyl und 1- oder 2-Aethoxyäthyl, Halogenniederalkyl, z.B. 2-Fluor-,

2-Chlor-, 2-Brom- und 2-Jodäthyl, Oxoniederalkyl, z.B. Acetonyl, gegebenenfalls geschütztes, z.B. acyliertes Hydroxyniederalkyl, z.B. 2-Hydroxyäthyl und Acetoxyäthyl, gegebenenfalls verestertes Carboxyniederalkyl, z.B. 2-Carboxymethyl und 2-Aethoxycarbonylmethyl, Amino- und Mono- oder Diniederalkylamino-niederalkyl, z.B. 2-Aminoäthyl, 2-Methylaminoäthyl, 2-Dimethylaminoäthyl und Acylaminoniederalkyl, z.B. 2-Acetamidoäthyl, Niederalkylphenyl, z.B. Tolyl und Xylyl, Niederalkoxyphenyl, z.B. Methoxyphenyl, Halogenphenyl, z.B. Fluor- oder Chlorphenyl, Hydroxyphenyl, Carboxyphenyl und Aminophenyl, wobei die Substituenten in o-, m- oder p-Stellung des Phenylkernes stehen können, Phenacetyl, und Heterocyclylniederalkyl, insbesondere Heterocyclylmethyl, worin Heterocyclyl beispielsweise gegebenenfalls z.B. durch Niederalkyl, Hydroxy, verestertes Hydroxy, Halogen, Sulfo, amidiertes Sulfo, Amino oder Aminomethyl substituiertes Furyl, Thienyl, Imidazolyl, Triazolyl, Tetrazolyl, Thiazolyl, Isothiazolyl, Thiadiazolyl, Thiatriazolyl, Oxazolyl, Isoxazolyl, Oxadiazolyl, Pyridyl, Pyridazinyl oder Pyrimidyl darstellt, beispielsweise 2-Furyl-, 5-Aminomethyl-2-furyl-, 2-Thienyl-, 5-Aminomethyl-2-thienyl-, 2-Imidazolyl-, 1,2,3-Triazol-4-yl-, 2-Amino-1,3-thiazol-5-yl-methyl, oder 5-Amino-1,2,4-thiadiazol-3-ylmethyl.

Eine organische Silyl- oder Stannylgruppe $R_1^a$ ist in erster Linie eine solche, worin das Silicium bzw. Zinnatom vorzugsweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Linie Triniederalkylsilyl, insbesondere Trimethylsilyl, ferner Dimethyl-tert.-butyl-silyl, Niederalkoxy-niederalkyl-halogen-silyl, z.B. Methoxy-methyl-chlor-silyl, oder Diniederalkyl-halogensilyl, z.B. Dimethyl-chlor-silyl, oder entsprechend substituiertes Stannyl, z.B. Tri-n-butylstannyl.

Eine veresterte Hydroxygruppe $R_1$ ist eine Gruppe $R_1^b$-O-, worin $R_1^b$ der Acylrest einer organischen Carbon- oder Sulfonsäure oder einer anorganischen Säure ist.

Der Acylrest $R_1^b$ einer organischen Carbonsäure hat bis zu 19 C-Atome, umfasst auch den Acylrest eines Halbesters der Kohlensäure und einer gegebenenfalls substituierten Carbaminsäure. Solche Acylreste haben beispielsweise die Formel H-CO-, $R_1^a$-CO-, Heterocyclyl-CO-, $R_1^a$-O-CO-, $H_2N$-CO- und $R_1^aR_1^a$N-CO-, worin $R_1^a$ und Heterocyclyl die oben gegebenen Bedeutungen haben.

Der Acylrest $R_1^b$ einer organischen Sulfonsäure hat beispielsweise die Formel $R_1^a$-$SO_2$-, worin $R_1^a$ die oben gegebenen Bedeutungen eines gegebenenfalls substituierten Kohlenwasserstoffrestes hat.

Der Acylrest $R_1^b$ einer anorganischen Säure ist beispielsweise derjenige einer sauerstoffhaltigen Schwefel- oder Phosphorsäure, z.B. $HOSO_2$- oder $H_2PO_3$-.

Bevorzugt ist $R_1$ Hydroxy, Niederalkoxy, z.B. Methoxy, Phenylniederalkoxy, z.B. Benzyloxy, oder auch Niederalkanoyloxy, z.B. Formyloxy oder Acetoxy, Aminothiazolylcarbonyloxy, z.B. 2-Amino-1,3-thiazol-4-ylcarbonyloxy, Aminothiadiazolylcarbonyloxy, z.B. 5-Amino-1,2,4-thiadiazol-3-ylcarbonyloxy, Niederalkoxycarbonyloxy, z.B. Methoxycarbonyloxy oder Aethoxycarbonyloxy, Carbamoyloxy, N-Mono- oder N,N-Diniederalkylcarbamoyloxy, z.B. N-Methylcarbamoyloxy oder N,N-Dimethylcarbamoyloxy, Niederalkansulfonyloxy, z.B. Methansulfonyloxy, Arensulfonyloxy, z.B. Benzolsulfonyloxy oder Toluolsulfonyloxy, oder Hydroxysulfonyloxy (oder ein Salz davon, z.B. das Natriumsalz).

In erster Linie ist $R_1$ Hydroxy.

Die in Verbindungen der Formel (I) vorhandenen funktionellen Gruppen, insbesondere Carboxyl- und Amino-, ferner Hydroxy- und Sulfogruppen, sind gegebenenfalls durch Schutzgruppen geschützt, die in der Penicillin-, Cephalosporin- und Peptidchemie verwendet werden.

Solche Schutzgruppen sind leicht, das heisst, ohne dass unerwünschte Nebenreaktionen stattfinden, beispielsweise solvolytisch, reduktiv, photolytisch oder auch unter physiologischen Bedingungen, abspaltbar.

Schutzgruppen dieser Art, sowie ihre Abspaltung sind beispielsweise beschrieben in "Protective Groups in Organic Chemistry", Plenum Press, London, New York, 1973, ferner in "The Peptides", Vol. I, Schröder and Lubke, Academic Press, London, New York, 1965, sowie in "Methoden der organischen Chemie", Houben-Weyl, 4. Auflage, Bd. 15./1, Georg Thieme Verlag, Stuttgart, 1974.

So sind Carboxylgruppen, z.B. die Carboxylgruppe $R_2$, üblicherweise in veresterter Form geschützt, wobei solche Estergruppierungen unter schonenden Bedingungen leicht spaltbar sind. In dieser Art geschützte Carboxylgruppen enthalten als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, Arylmethoxycarbonyl mit einem oder zwei Arylresten, wobei diese gegebenenfalls, z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls,z.B. wie oben erwähnt, substituiertes Benzyloxycarbonyl, z.B. 4-Nitro-benzyloxycarbonyl oder 4-Methoxy-benzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyäthoxycarbonyl oder 1-Aethoxymethoxycarbonyl, 1-Niederalkylthioniederalkoxycarbonyl, wie 1-Methylthiomethoxycarbonyl oder 1-Aethylthioäthoxycarbonyl, Aroylmethoxycarbonyl, worin die Aroylgruppe gegebenenfalls, z.B. durch Halogen, wie Brom, substituiertes

Benzoyl darstellt, z.B. Phenacyloxycarbonyl, 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder 2-(trisubstituiertes Silyl)-äthoxycarbonyl, worin die Substituenten unabhängig voneinander je einen gegebenenfalls substituierten, z.B. durch Niederalkyl, Niederalkoxy, Aryl, Halogen und/oder Nitro substituierten, aliphatischen, araliphatischen, cycloaliphatischen oder aromatischen Kohlenwasserstoffrest mit z.B. bis zu 15 Kohlenstoffatomen, wie entsprechendes, gegebenenfalls substituiertes Niederalkyl, Phenylniederalkyl, Cycloalkyl oder Phenyl bedeuten, z.B. 2-Triniederalkylsilyläthoxycarbonyl, wie 2-Trimethylsilyläthoxycarbonyl oder 2-(Di-n-butyl-methyl-silyl)-äthoxycarbonyl, oder 2-Triarylsilyläthoxycarbonyl, wie 2-Triphenylsilyläthoxycarbonyl.

Weitere, in veresterter Form vorliegende geschützte Carboxylgruppen sind entsprechende Silyloxycarbonyl-, insbesondere organische Silyloxycarbonylgruppen, ferner entsprechende Stannyloxycarbonylgruppen. In diesen enthält das Silicium- bzw. Zinnatom vorzugsweise Niederalkyl, insbesondere Methyl, ferner Niederalkoxy, z.B. Methoxy, und/oder Halogen, z.B. Chlor, als Substituenten. Geeignete Silyl- bzw. Stannylschutzgruppen sind in erster Linie Triniederalkylsilyl, insbesondere Trimethylsilyl, ferner Dimethyl-tert.butylsilyl, Niederalkoxyniederalkyl-halogen-silyl, z.B. Methoxy-methyl-chlor-silyl, oder Diniederalkyl-halogen-silyl, z.B. Dimethyl-chlor-silyl, oder entsprechend substituierte Stannylgruppen, z.B. Tri-n-butylstannyl.

Bevorzugte geschützte Carboxylgruppen sind tert.-Niederalkoxycarbonyl, wie tert.-Butyloxycarbonyl, und in erster Linie gegebenenfalls, z.B. wie oben erwähnt, substituiertes Benzyloxycarbonyl, wie 4-Nitro-benzyloxycarbonyl, oder Diphenylmethoxycarbonyl.

Eine unter physiologischen Bedingungen spaltbare, veresterte Carboxylgruppe ist in erster Linie eine Acyloxymethyloxycarbonylgruppe, worin Acyl z.B. den Rest einer organischen Carbonsäure, in erster Linie einer gegebenenfalls substituierten Niederalkancarbonsäure bedeutet, oder worin Acyloxymethyl den Rest eines Lactons bildet, oder auch 1-Niederalkyloxycarbonyloxy-niederalkyloxycarbonyl, worin Niederalkyl Methyl, Propyl, Butyl oder insbesondere Aethyl ist. Solche Gruppen sind Niederalkanoyloxymethoxycarbonyl, z.B. Acetyloxymethyloxycarbonyl oder Pivaloyloxymethoxycarbonyl, Aminoniederalkanoylmethoxycarbonyl, insbesondere α-Amino-niederalkanoyloxymethoxycarbonyl, z.B. Glycyloxymethoxycarbonyl, L-Valyloxymethoxycarbonyl, L-Leucyloxymethoxycarbonyl, Phthalidyloxycarbonyl, z.B. 2-Phthalidyloxycarbonyl, 4-Crotonolactonyl oder gamma-Butyrolacton-4-yl, Indanyloxycarbonyl, z.B. 5-Indanyloxycarbonyl oder 1-Aethyloxycarbonyloxyäthyloxycarbonyl.

Eine geschützte Aminogruppe kann z.B. in Form einer leicht spaltbaren Acylamino-, Arylmethyl-amino-, verätherten Mercaptoamino- 2-Acyl-niederalk-1-en-yl-amino-, Silyl- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogen-niederalkanoyl, wie 2-Halogenacetyl, insbesondere 2-Chlor-, 2-Brom-, 2-Jod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.-Niederalkyl, wie tert.-Butyl, Niederalkoxy, wie Methoxy, Hydroxy, Halogen, z.B. Chlor und/oder Nitro, mono- oder polysubstituiertes Phenyl darstellen, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogen-niederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder 2-(trisubstituiertes Silyl)-äthoxycarbonyl, worin die Substituenten unahängig voneinander je einen gegebenenfalls substituierten, z.B. durch Niederalkyl, Niederalkoxy, Aryl, Halogen oder Nitro, substituierten, aliphatischen, araliphatischen, cycloaliphatischen oder aromatischen Kohlenwasserstoffrest mit z.B. bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilyläthoxycarbonyl, wie 2-Trimethylsilyläthoxycarbonyl oder 2-(Di-n-butyl-methyl-silyl)-äthoxycarbonyl, oder 2-Triarylsilyläthoxycarbonyl, wie 2-Triphenylsilyläthoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diäthylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenenfalls, z.B. durch Nitro substituiertes Diphenylniederalkylphosphoryl, z.B. Dibenzylphosphoryl oder Di-4-nitrobenzylphosphoryl,

gegebenenfalls substituiertes Phenyloxy-phenyl-phosphonyl, z.B. Phenyloxy-phenyl-phosphonyl, Diniederalkylphosphinyl, z.B. Diäthylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylaminogruppe darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor und/odet Nitro substituiertes Phenyl ist. Eine entsprechende Aminoschutzgruppe ist z.B. 4-Nitrophenylthio.

In einem als Aminoschutzgruppe verwendbaren 1-Acyl-niederalk-1-en-2-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor und/oder Nitro substituierten Benzoesäure, oder insbesondere eines Kohlensäurehalbesters, wie eines Kohlensäure-niederalkylhalbesters. Entprechende Schutzgruppen sind in erster Linie 1-Niederalkanoylprop-1-en-2-yl, z.B. 1-Acetyl-prop-1-en-2-yl, oder 1-Niederalkoxycarboyl-prop-1-en-2-yl, z.B. 1-Aethoxycarbonyl-prop-1-en-2-yl.

Eine Silyl- oder Stannylaminogruppe ist in erster Linie eine organische Silyl- bzw. Stannylaminogruppe, worin das Silicium- bzw. Zinnatom vorzugsweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Linie Triniederalkylsilyl, insbesondere Trimethylsilyl, ferner

Dimethyl-tert.-butyl-silyl, Niederalkoxy-niederalkyl-halogen-silyl, z.B. Methoxy-methyl-chlor-silyl, oder Diniederalkyl-halogensilyl, z.B. Dimethyl-chlor-silyl, oder entsprechend substituiertes Stannyl, z.B. Tri-n-butylstannyl.

Eine Aminogruppe kann auch in protonierter Form geschützt werden; als entsprechende Anionen kommen in erster Linie diejenigen von starken anorganischen Säuren, wie von Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, gegebenenfalls z.B. wie angegeben, substituiertes Benzyloxycarbonyl, z.B. 4-Nitrobenzyloxycarbonyl, oder Diphenylmethoxycarbonyl, oder 2-Halogenniederalkoxycarbonyl, wie 2,2,2-Trichloräthoxycarbonyl, ferner Trityl oder Formyl.

Hydroxyschutzgruppen sind z.B. Acylreste, wie gegebenenfalls z.B. durch Halogen, substituiertes Niederalkanoyl, wie 2,2-Dichloracetyl, oder insbesondere die im Zusammenhang mit einer geschützten Aminogruppe genannten Acylreste von Kohlensäurehalbestern, insbesondere 2,2,2-Trichloräthoxycarbonyl, oder die organischen Silyl- oder Stannylreste $R_1^a$, ferner leicht abspaltbare veräthernde Gruppen, wie tert.-Niederalkyl, z.B. tert.-Butyl, 2-Halogenniederalkyl z.B. 2,2,2-Trichlor-, 2-Chlor-, 2-Brom- oder 2-Jodäthyl, 2-oxa- oder 2-thiaaliphatische oder -cycloaliphatische Kohlenwasserstoffreste, in erster Linie 1-Niederalkoxy-niederalkyl oder 1-Niederalkylthioniederalkyl, z.B. Methoxymethyl, 1-Methoxyäthyl, 1-Aethoxy-äthyl, 1-Methylthiomethyl, 1-Methylthioäthyl oder 1-Aethylthioäthyl, oder 2-Oxa- oder 2-Thiacycloalkyl mit 5-7 Ringatomen, z.B. 2-Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Eine geschützte Sulfogruppe ist in erster Linie eine veresterte, wie mit einem aliphatischen, cycloaliphatischen, cycloaliphatisch-aliphatischen, aromatischen oder araliphatischen Alkohol, z.B. einem Niederalkanol, oder mit einem Silyl- oder Stannylrest, wie Triniederalkylsilyl, veresterte Sulfogruppe. In einer Sulfogruppe kann die Hydroxygruppe beispielsweise, wie die Hydroxygruppe in einer veresterten Carboxygruppe veräthert sein.

Salze von erfindungsgemässen Verbindungen sind in erster Linie pharmazeutisch verwendbare, nicht toxische Salze, wie diejenigen von Verbindungen der Formel I mit sauren Gruppen, z.B. mit einer freien Carboxyl- oder Sulfogruppe. Solche Salze sind in erster Linie Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wobei in erster Linie aliphatische, cycloaliphatische, cycloaliphatischaliphatische oder araliphatische primäre, sekundäre oder tertiäre Mono-, Di- oder Polyamine, sowie heterocyclische Basen für die Salzbildung in Frage kommen, wie Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-2-diäthylaminoäthylester, Niederalkylenamine, z.B. 1-Aethyl-piperidin, Cycloalkylamine, z.B. Dicyclohexylamin, oder Benzylamine, z.B. N,N'-Dibenzyläthylendiamin, ferner Basen vom Pyridintyp, z.B. Pyridin, Collidin oder Chinolin. Verbindungen der Formel I mit einer basischen Gruppe können Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin, bilden. Bei Anwesenheit von mehreren sauren oder basischen Gruppen können Mono- oder Polysalze gebildet werden. Verbindungen der Formel I mit einer sauren, z.B.

freien Carboxylgruppe, und einer freien basischen Gruppe, z.B. einer Aminogruppe, können auch in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung können auch pharmazeutisch ungeeignete Salze Verwendung finden. Zur therapeutischen Anwendung gelangen nur die pharmazeutisch verwendbaren, nicht toxischen Salze, die deshalb bevorzugt werden.

Die Verbindungen der Formel (I) haben in 3- und 5-Stellung die R-Konfiguration und in 6-Stellung die R- oder S-Konfiguration oder können auch Mischungen der 6R- und 6S-Konfiguration darstellen. Verbindungen mit 6R-Konfiguration (worin sich der Substituent $R_1-CH_2-$ in β-Stellung befindet und die beiden Wasserstoffatome in 5- und 6-Stellung cis zueinander stehen) sind bevorzugt.

Die Erfindung betrifft insbesondere Verbindungen der Formel (I), worin funktionelle Gruppen entweder nicht oder in physiologisch spaltbarer Form geschützt sind, und deren pharmazeutisch annehmbaren Salze, da hauptsächlich diese Verbindungen die angegebene Wirksamkeit besitzen und für den angegebenen Zweck benutzt werden können, während die geschützten Verbindungen hauptsächlich als Zwischenprodukte dienen.

Hervorzuheben sind Verbindungen der Formel (I), worin $R_1$ Hydroxy, Niederalkoxy, z.B. Methoxy, Phenylniederalkoxy, z.B. Benzyloxy, Niederalkanoyloxy, z.B. Formyloxy oder Acetoxy, Aminothiazolylcarbonyloxy, z.B. 2-Amino-1,3-thiazol-4-ylcarbonyloxy, Aminothiadiazolylcarbonyloxy, z.B. 5-Amino-1,2,4-thiadiazol-3-ylcarbonyloxy, Niederalkoxycarbonyloxy, Carbamoyloxy, N-Mono- oder N,N-Diniederalkylcarbamoyloxy, z.B. N-Methylcarbamoyloxy oder N,N-Dimethylcarbamoyloxy, Niederalkansulfonyloxy, z.B. Methansulfonyloxy, Arensulfonyloxy, z.B. Benzolsulfonyloxy oder Toluolsulfonyloxy, oder Hydroxysulfonyloxy (oder ein Salz davon, z.B. das Natriumsalz), darstellt und

$R_2$ Carboxy oder in physiologisch spaltbarer Form verestertes Carboxy, z.B.Niederalkanoyloxymethoxycarbonyl, z.B. Acetyloxymethyloxycarbonyl oder Pivaloyloxymethoxycarbonyl, Aminoniederalkanoylmethoxy, insbesondere α-Amino-niederalkanoyloxymethoxycarbonyl, z.B. Glycyloxymethoxycarbonyl, L-Valyloxymethoxycarbonyl, L-Leucyloxymethoxycarbonyl, Phthalidyloxycarbonyl, z.B. 2-Phthalidyloxycarbonyl, 4-Crotonolactonyl oder gamma-Butyrolacton-4-yl, Indanyloxycarbonyl, z.B. 5-Indanyloxycarbonyl oder 1-Aethyloxycarbonyloxyäthyloxycarbonyl bedeutet, sowie die pharmazeutisch annehmbaren Salze von solchen Verbindungen, die eine salzbildende Gruppe enthalten, z.B. die Alkalimetallsalze, z.B. die Natriumsalze dieser Verbindungen, worin $R_2$ Carboxy bedeutet.

Die Erfindung betrifft insbesondere die Verbindungen der Formel (I), worin $R_1$ Hydroxy, Methoxy oder Benzyloxy darstellt und $R_2$ Carboxy bedeutet, sowie die pharmazeutisch annehmbare Salze, z.B. die Natriumsalze davon.

Die Erfindung betrifft in erster Linie die in den Beispielen beschriebenen Verbindungen, insbesondere die Verbindung der Formel (I), worin $R_1$ Hydroxy darstellt und $R_2$ Carboxy bedeutet, sowie die pharmazeutisch annehmbaren Salze davon, z.B. das Natriumsalz.

Die Verbindungen der Formel (I) und Salze davon werden auf an sich bekannte Weise hergestellt.

Verbindungen der Formel (I) und Salze von solchen Verbindungen, die eine salzbildende Gruppe aufweisen, werden beispielsweise hergestellt, indem man

a) eine Verbindung der Formel

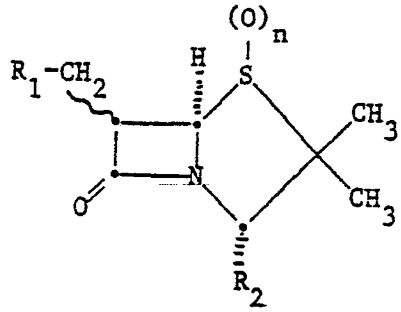

(IIa),

worin $R_1$ und $R_2$ die unter Formel (I) genannten Bedeutungen haben und der Index n den Wert 0 oder 1 hat, in 1-Stellung oxidiert, oder

b) in eine Verbindung der Formel

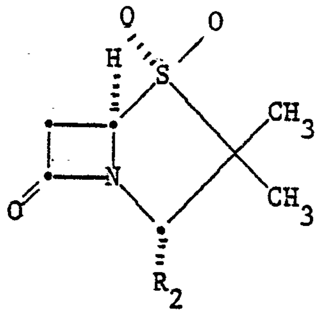

(IIb),

worin $R_2$ eine geschützte Carboxylgruppe darstellt, die Gruppe $R_1$-$CH_2$- einführt, oder

c) in einer Verbindung der Formel

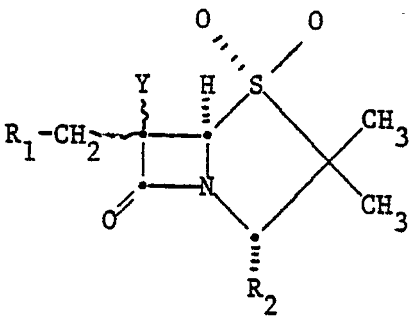

(IIc),

worin $R_1$ und $R_2$ die unter Formel (I) genannten Bedeutungen haben und Y eine in ein Wasserstoffatom überführbare Gruppe darstellt, die Gruppe Y in ein Wasserstoffatom überführt, und, wenn erwünscht oder nodwendig, in einer erhältlichen Verbindung eine Gruppe $R_1$ in eine andere Gruppe $R_1$ überführt, und/oder eine Gruppe $R_2$ in eine andere Gruppe $R_2$ überführt, und/oder ein erhältliches Salz in die freie Verbindung oder in ein anderes Salz überführt, und/oder eine erhältliche freie Verbindung mit einer salzbildenden Gruppe in ein Salz überführt, und/oder ein erhältliches Gemisch von isomeren Verbindungen der Formel (I) in die einzelnen Isomeren auftrennt.

a) Oxidation der 1-Stellung

Die Oxidation von Verbindungen der Formel (IIa) erfolgt durch Behandlung mit Sulfid- oder Sulfoxidgruppen in Sulfongruppen überführenden Mitteln, insbesondere mit Wasserstoffperoxid, organischen Persäuren, insbesondere aliphatischen Percarbonsäuren, z.B. Peressigsäure, Perbenzoesäure, Chlorperbenzoesäure, z.B. m-Chlorperbenzoesäure, oder Monoperphthalsäure, mit oxidierenden anorganischen Säuren oder deren Salzen, z.B. Salpetersäure, Chromsäure, Kaliumpermanganat, oder einem Alkalimetallhypochlorit, z.B. Natriumhypochlorit, sowie auch durch anodische Oxidation. Die Oxidation wird bevorzugt in einem geeigneten inerten Lösungsmittel, beispielsweise einem Halogenkohlenwasserstoff, z.B. Methylenchlorid, Chloroform oder Tetrachlorkohlenstoff, einem Alkohol, z.B. Methanol oder Aethanol, einem Keton, z.B. Aceton, einem Aether, z.B. Diäthyläther, Dioxan oder Tetrahydrofuran, einem Amid, z.B. Dimethylformamid, einem Sulfon, z.B. Dimethylsulfon, einer flüssigen organischen Carbonsäure, z.B. Essigsäure, oder in Wasser oder einem Gemisch dieser Lösungsmittel, insbesondere einem wasserhaltigen Gemisch, z.B. wässriger Essigsäure, bei Raumtemperatur, oder unter Kühlen oder leichtem Erwärmen, d.h. bei etwa -20 bis etwa + 90°, bevorzugt bei etwa +18 bis etwa +30°, durchgeführt. Die Oxidation kann auch stufenweise durchgeführt werden, indem zunächst bei niederer Temperatur, d.h. bei etwa -20 bis etwa 0° bis zur Sulfoxidstufe oxidiert wird, das gegebenenfalls isoliert wird, worauf in einem zweiten Schritt, bevorzugt bei höherer Temperatur, etwa bei Raumtemperatur, das Sulfoxid zum Sulfon, d.h. dem 1,1-Dioxid der Formel (I), oxidiert wird.

Zur Aufarbeitung kann gegebenenfalls noch vorhandenes überschüssiges Oxidationsmittel durch Reduktion, insbesondere durch Behandeln mit einem Reduktionsmittel, wie einem Thiosulfat, z.B. Natriumthiosulfat, zerstört werden.

b) Einführung einer Gruppe $R_1$-$CH_2$-: Die Gruppe $R_1$-$CH_2$- wird durch eine Alkylierungsreaktion eingeführt, beispielsweise, indem man ein Mono- oder Dianion einer Verbindung der Formel (IIb) mit Formaldehyd oder einem reaktionsfähigen funktionellen Derivat davon umsetzt und anschliessend das Reaktionsprodukt mit einer Protonenquelle behandelt.

Monoanionen von Verbindungen der Formel (IIb) haben in 6-Stellung, Dianionen in 6- und in 3-Stellung anstelle eines Wasserstoffatoms eine negative Ladung. Solche Verbindungen werden üblicherweise in situ hergestellt, beispielsweise, indem man eine Verbindung der Formel (IIb) mit einem oder zwei Mol eines Metallierungsreagenses umsetzt. Geeignete Metallierungsreagentien sind substituierte und unsubstituierte Alkalimetallamide, Alkalimetallhydride oder Alkalimetallniederalkylverbindungen, worin das Alkalimetall Natrium oder insbesondere Lithium ist, z.B. Natrium- oder Lithiumamid, Lithium-bis-trimethylsilylamid, Natriumhydrid, Lithiumhydrid und bevorzugt Lithiumdiisopropylamid und Butyllithium. Eine weitere Methode zur Herstellung von Monoanionen besteht darin, dass man eine 6-Halogenverbindung einer Verbindung der Formel (IIb), worin die 6-Stellung durch Halogen, z.B. Chlor, Brom oder insbesondere Jod substituiert ist, mit Magnesium oder einem Grignardreagens, z.B. mit einem Niederalkylmagnesiumhalogenid, z.B. Methylmagnesiumbromid, oder mit aktiviertem Zink oder Zinkamalgam umsetzt.

Reaktionsfähige funktionelle Derivate des Formaldehydes sind beispielsweise Paraformaldehyd und Verbindungen der Formel $R_1$-$CH_2$-X, worin $R_1$ eine verätherte oder veresterte Hydroxygruppe ist und X eine nukleofuge Abgangsgruppe , beispielsweise ein Halogenatom, z.B. Chlor, Brom oder Jod, eine Sulfonyloxygruppe, z.B. Mesyloxy oder Tosyloxy, oder auch eine Gruppe $R_1^a$-O-, darstellt.

 

Die Herstellung des Mono- und Dianions erfolgt in einem inerten, nicht-protischen Lösungsmittel, beispielsweise in einem Kohlenwasserstoff, z.B. Hexan, Benzol, Toluol oder Xylol, einem schwach polaren Aether, z.B. Diäthyläther, Tetrahydrofuran oder Dioxan, oder einem Säureamid, z.B. Hexamethylphosphorsäuretriamid, oder Mischungen davon, bei Temperaturen zwischen etwa -80° und etwa Raumtemperatur, wobei die Herstellung mittels Metallierungsreagentien, Magnesium oder Grignardreagentien, bevorzugt bei niederen Temperaturen, etwa -70° bis etwa -30° erfolgt, während die Herstellung mittels Zink, bevorzugt bei etwa 0° bis 30°, insbesondere bei Raumtemperatur erfolgt.

Der Umsatz des Mono- oder Dianions mit Formaldehyd oder dem reaktionsfähigen funktionellen Derivat davon findet in dem gleichen Lösungsmittel oder Lösungsmittelgemisch und unter den gleichen obigen Temperaturbedingungen statt. Das Mono- oder Dianion kann auch in Gegenwart des Formaldehyds oder des reaktionsfähigen Derivates davon gebildet werden.

In einer bevorzugten Ausführungsform wird gasförmiger, trockener Formaldehyd bei etwa -78° in eine Monoanionen enthaltende Reaktionsmischung, die bevorzugt durch Umsatz einer 6-Halogen-, insbesondere 6-Brom- oder 6-Jod-substituierten Verbindung der Formel IIb mit einem Niederalkylmagnesiumhalogenid, insbesondere Methylmagnesiumjodid, in einem Aether, insbesondere Tetrahydrofuran, erhalten wird, geleitet.

Nach der Alkylierungsreaktion wird das Reaktionsprodukt

mit einer Protonenquelle behandelt, z.B. mit Wasser, einem Alkohol, z.B. Methanol oder Aethanol, einer organischen oder anorganischen Säure, z.B. Essigsäure, Salzsäure, Schwefelsäure oder einer ähnlichen Protonen abgebenden Verbindung, bevorzugt wiederum bei niederen Temperaturen.

c) Austausch von Y gegen Wasserstoff: In einer Verbindung der Formel (IIc) ist Y als eine durch Wasserstoff austauschbare Gruppe, beispielsweise eine Isonitrilgruppe C=N- oder Halogen, z.B. Chlor, Jod oder insbesondere Brom.

Der Austausch von Y gegen ein Wasserstoffatom erfolgt durch Behandlung mit einem geeigneten Reduktionsmittel. Geeignete Reduktionsmittel sind beispielsweise Metallhydride, insbesondere Zinnhydride wie Triniederalkylzinnhydride oder Diniederalkylzinnhydride, insbesondere Tri-n-butylzinnhydrid, oder auch Triphenylzinnhydrid, Lithiumaluminiumhydrid oder Natriumborhydrid, Wasserstoff in statu nascendi, z.B. erzeugt aus einem Metall und einer Protonenquelle, insbesondere Zink und Essigsäure, oder Natrium und Alkohol, z.B. Amylalkohol, oder auch katalytisch, z.B. durch Palladium-Kohle aktivierter Wasserstoff.

Die Reduktion findet in einem geeigneten, gegebenenfalls inerten Lösungsmittel statt. Metallhydride werden beispielsweise in einem Kohlenwasserstoff, z.B. Pentan, Hexan, Benzol, Toluol oder Xylol, angewendet, während die Reduktion mit Wasserstoff in statu nascendi in der benutzten Protonenquelle, die gegebenenfalls durch ein inertes Lösungsmittel verdünnt sein kann, durchgeführt wird. In einem der genannten Lösungsmittel oder Lösungsmittelgemische kann auch die katalytische Reduktion stattfinden. Je nach angewandtem Reduktionsmittel arbeitet man bei Raumtemperatur oder erniedrigter oder erhöhter Temperatur etwa bei -80 bis +100°.

Ein bevorzugtes Reduktionsverfahren ist die Behandlung einer

Verbindung der Formel(IIc), worin Y eine Isonitrilgruppe oder Brom ist, mit Tri-n-butylzinnhydrid in einem inerten Lösungsmittel, z.B. Benzol, bei Raumtemperatur bis zur Siedetemperatur des verwendeten Lösungsmittels, d.h. bis etwa 80°, gegebenenfalls in Gegenwart katalytischer Mengen Azobisisobutyronitril (D.I. John, E.J. Thomas und N.D. Tyrell, J.C.S. Chem. Comm. 1979, S. 345; J.A. Aimetti, E.S. Hamanaka, D.A. Johnson und M.S. Kellogg, Tetrahedron Letters, No. 48, 1979, S. 4631).

In einer erhältlichen Verbindung der Formel (I) können die Substituenten $R_1$ und $R_2$ im Rahmen ihrer Bedeutungen und in beliebiger Reihenfolge in andere Substituenten $R_1$ oder $R_2$ übergeführt werden. So kann eine Hydroxygruppe $R_1$ durch Verätherung oder Veresterung in eine verätherte bzw. veresterte Hydroxygruppe $R_1^a$-O- bzw- $R_1^b$-O- übergeführt werden. Eine freie Carboxylgruppe $R_2$ kann verestert und eine veresterte Carboxylgruppe $R_2$ kann in eine freie Carboxylgruppe übergeführt werden. Gegebenenfalls im Rest $R_1$ vorhandene Schutzgruppen können ebenfalls abgespalten werden. Diese nachträglichen Operationen werden in an sich bekannter Weise durchgeführt, beispielweise wie folgt:

<u>Verätherung und Veresterung einer Hydroxygruppe $R_1$:</u> In einer Verbindung der Formel (I), worin die Gruppe $R_2$ als funktionell abgewandelte Carboxylgruppe vorliegt, und andere, gegebenenfalls vorhandene funktionelle Gruppen vorzugsweise geschützt sind, kann eine Hydroxygruppe $R_1$ in an sich bekannter Weise in eine verätherte Hydroxygruppe $R_1^a$-O- übergeführt werden.

Geeignete Verätherungsmittel sind beispielsweise Diazoverbindungen $R_1^aN_2$, wie gegebenenfalls substituierte Diazoniederalkane, z.B. Diazomethan, Diazoäthan, Diazo-n-butan oder Diphenyldiazomethan. Diese Reagentien werden in Gegenwart eines geeigneten inerten Lösungsmittels, wie eines aliphatischen, cycloaliphatischen

oder aromatischen Kohlenwasserstoffs, wie Hexan, Cyclohexan, Benzol oder Toluol, eines halogenierten aliphatischen Kohlenwasserstoffs, z.B. Methylenchlorid, oder eines Aethers, wie eines Diniederalkyläthers, z.B. Diäthyläther, oder eines cyclischen Aethers, z.B. Tetrahydrofuran oder Dioxan, oder eines Lösungsmittelgemisches, und, je nach Diazoreagens, unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre, zur Anwendung gebracht.

Weitere geeignete Verätherungsmittel sind Ester von Alkoholen $R_1^a$-OH, in erster Linie solche mit starken anorganischen oder organischen Säuren, wie Mineralsäuren, z.B. Halogenwasserstoffsäuren, wie Chlorwasserstoff-, Bromwasserstoff- oder Jodwasserstoffsäure, ferner Schwefelsäure, oder Halogen-schwefelsäuren, z.B. Fluorschwefelsäure, oder starken organischen Sulfonsäuren, wie gegebenenfalls, z.B. durch Halogen, wie Fluor, substituierten Niederalkansulfonsäuren, oder aromatischen Sulfonsäuren, wie z.B. gegebenenfalls durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure. Solche Ester sind z.B. $R_1^a$-Halogenide, u.a. Niederalkylhalogenide, Di-$R_1^a$-sulfate, z.B. Diniederalkylsulfate, wie Dimethylsulfat, ferner Fluorsulfonsäure-$R_1^a$-ester, wie -niederalkylester, z.B. Fluorsulfonsäuremethylester, oder gegebenenfalls Halogen-substituierte Methansulfonsäure-$R_1^a$-ester, wie -niederalkylester, z.B. Trifluormethansulfonsäuremethylester. Sie werden üblicherweise in Gegenwart eines inerten Lösungsmittels, wie eines gegebenenfalls halogenierten, wie chlorierten, aliphatischen, cycloaliphatischen oder aromatischen Kohlenwasserstoffs, z.B. Methylenchlorid, eines Aethers, wie Dioxan oder Tetrahydrofuran, oder eines Gemisches verwendet. Dabei wendet man vorzugsweise geeignete Kondensationsmittel, wie Alkalimetallcarbonate oder -hydrogencarbonate, z.B. Natrium- oder Kaliumcarbonat oder -hydrogencarbonat (üblicherweise zusammen mit einem Sulfat), oder organische Basen, wie, üblicherweise sterisch gehinderte, Triniederalkylamine, z.B. N,N-Diisopropyl-N-äthyl-amin (vorzugsweise zusammen mit Halogensulfonsäureniederalkylestern oder gegebenenfalls halogensubstituierten Methansulfonssäure-niederalkylestern) an, wobei unter Kühlen, bei Raumtemperatur oder unter Erwärmen, z.B. bei Temperaturen von etwa -20°C bis etwa 50°C und, wenn notwendig, in einem geschlossenen Gefäss und/oder in einer Inertgas-, z.B. Stickstoffatmosphäre, gearbeitet wird.

Durch Phasentransfer-Katalyse (siehe Dehmlow, Angewandte Chemie, Bd. 5, S. 187 (1974), kann die oben beschriebene Verätherungsreaktion wesentlich beschleunigt werden. Als Phasentransfer-Katalysatoren können quartäre Phosphoniumsalze und insbesondere quartäre Ammoniumsalze, wie gegebenenfalls substituierte Tetraalkylammoniumhalogenide, z.B. Tetrabutylammoniumchlorid, -bromid oder -jodid, oder auch Benzyl-triäthylammoniumchlorid, in katalytischen oder bis zu äquimolaren Mengen verwendet werden. Als organische Phase kann irgendeines der mit Wasser nicht mischbaren Lösungsmittel dienen, beispielsweise einer der gegebenenfalls halogenierten, wie chlorierten niederaliphatischen, cycloaliphatischen oder aromatischen Kohlenwasserstoffe, wie Tri- oder Tetrachloräthylen, Tetrachloräthan, Tetrachlorkohlenstoff, Chlorbenzol, Toluol oder Xylol. Die als Kondensationsmittel geeigneten Alkalimetallcarbonate oder -hydrogencarbonate, z.B. Kalium- oder Natriumcarbonat oder -hydrogencarbonat, Alkalimetallphosphate, z.B. Kaliumphosphat, und Alkalimetallhydroxide, z.B. Natriumhydroxid, können bei basenempfindlichen Verbindungen der Reaktionsmischung titrimetrisch, z.B. mittels eines Titrierautomaten, zugesetzt werden, damit der pH-Wert während der Verätherung zwischen etwa 7 und etwa 8,5 bleibt.

Weitere Verätherungsmittel sind geeignete Acetal-Verbindungen, wie gem-Di-$R_1^a$O-niederalkane, z.B. gem-Di-niederalkoxyniederalkane, wie 2,2-Dimethoxy-propan, die in Gegenwart von starken organischen Sulfonsäuren, wie p-Toluolsulfonsäure, und eines geeigneten Lösungsmittels, wie eines Diniederalkyl- oder Niederalkylensulfoxids, z.B. Dimethylsulfoxid, zur Anwendung gelangen, oder geeignete $R_1^a$ enthaltende Orthoester, z.B. Orthoameisensäure-tri-$R_1^a$-ester, z.B. -triniederalkylester, z.B. Orthoameisensäure-triäthylester, die in Gegenwart einer starken Mineralsäure, z.B. Schwefelsäure, oder einer starken organischen Sulfonsäure, wie p-Toluolsulfonsäure, und eines geeigneten Lösungsmittels, wie eines Aethers, z.B. Dioxan, verwendet werden.

Weitere Verätherungsmittel sind entsprechende trisubstituierte Oxoniumsalze (sogenannte Meerweinsalze), oder disubstituierte Carbenium- oder Haloniumsalze, worin die Substituenten die veräthernden Reste $R_1^a$ sind, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate oder Hexachlorantimonate. Solche Reagentien sind z.B. Trimethyloxonium- oder Triäthyloxonium-hexafluorantimonat, -hexachlorantimonat, -hexafluorphosphat oder -tetrafluorborat, Dimethoxycarbeniumhexafluorphosphat oder Dimethylbromoniumhexafluorantimonat. Man verwendet diese Verätherungsmittel vorzugsweise in einem inerten Lösungsmittel, wie einem Aether oder einem halogenierten Kohlenwasserstoff, z.B. Diäthyläther, Tetrahydrofuran oder Methylenchlorid, oder in einem Gemisch davon, wenn notwendig, in Gegenwart einer Base, wie einer organischen Base, z.B. eines vorzugsweise sterisch gehinderten Triniederalkylamins, z.B. N,N-Diisopropyl-N-äthyl-amin, und unter Kühlen, bei Raumtemperatur oder unter leichtem Erwärmen, z.B. bei etwa -20° bis etwa 50°C, wenn notwendig, in einem geschlossenen Gefäss und/oder in einer Inertgas-, z.B. Stickstoffatmosphäre.

Weitere Verätherungsmittel sind schliesslich entsprechende 1-substituierte 3-Aryltriazenverbindungen, worin der Substituent den veräthernden Rest $R_1^a$, und Aryl vorzugsweise gegebenenfalls substituiertes Phenyl, z.B. Niederalkylphenyl, wie 4-Methylphenyl, bedeutet. Solche Triazenverbindungen sind 3-Aryl-1-niederalkyl-triazene, z.B. 3-(4-Methylphenyl)-1-methyl-triazen, 3-(4-Methylphenyl)-1-äthyl-triazen oder 3-(4-Methylphenyl)-1-isopropyl-triazen. Diese Reagentien werden üblicherweise in Gegenwart von inerten Lösungsmitteln, wie gegebenenfalls halogenierten Kohlenwasserstoffen oder Aethern, z.B. Benzol, oder Lösungsmittelgemischen, und unter Kühlen bei Raumtemperatur und vorzugsweise bei erhöhter Temperatur, z.B. bei etwa 20° bis etwa 100°C, wenn notwendig, in einem geschlossenen Gefäss und/oder in einer Inertgas-, z.B. Stickstoffatmosphäre verwendet.

In einer erhältlichen Verbindung der Formel (I), worin die Gruppe $R_2$ als funktionell abgewandeltes Carboxyl vorliegt, und andere, gegebenenfalls vorhandene funktionelle Gruppen vorzugsweise geschützt sind, kann eine Hydroxygruppe $R_1$ in an sich bekannter Weise verestert werden. So kann man die Hydroxygruppe durch Behandeln einer Hydroxymethylverbindung der Formel (I) mit einem, den gewünschten Acylrest $R_1^b$ einer organischen Carbonsäure einführenden Acylierungsmittel in eine Acyloxygruppe $R_1^b$-O- umwandeln. Dabei verwendet man die $R_1^b$ entsprechende Carbonsäure oder ein reaktionsfähiges Derivat davon, insbesondere ein Anhydrid, inkl. ein gemischtes oder inneres Anhydrid einer solchen Säure. Gemischte Anhydride sind z.B. diejenigen mit Halogenwasserstoffsäuren, d.h. die entsprechenden Säurehalogenide, insbesondere -chloride, ferner mit Cyanwasserstoffsäure, oder dann diejenigen mit geeigneten Kohlensäurehalbderivaten, wie entsprechenden -halbestern (wie die z.B. mit einem Halogen-ameisensäure-niederalkyl-, wie Chlorameisensäure-äthylester oder -isobutylester, gebildeten gemischten Anhydride) oder mit gegebenenfalls substituierten, z.B. Halogen, wie Chlor, enthaltenden

Niederalkancarbonsäuren (wie die mit Pivalinsäurechlorid oder Trichloressigsäurechlorid gebildeten gemischten Anhydride). Innere Anhydride sind z.B. diejenigen von organischen Carbonsäuren, d.h. Ketene, wie Keten oder Diketen, oder diejenigen von Carbamin- oder Thiocarbaminsäuren, d.h. Isocyanate oder Isothiocyanate. Weitere reaktionsfähige, als Acylierungsmittel verwendbare Derivate von organischen Carbonsäuren sind aktivierte Ester, wie geeignet substituierte Niederalkyl-, z.B. Cyanmethylester, oder geeignet substituierte Phenyl-, z.B. Pentachlorphenyl- oder 4-Nitrophenylester. Die Veresterung kann, wenn notwendig, in Gegenwart von geeigneten Kondensationsmitteln, bei Verwendung von freien Carbonsäuren z.B. in Gegenwart von Carbodiimidverbindungen, wie Dicyclohexylcarbodiimid, oder Carbonylverbindungen, wie Diimidazolylcarbonyl, und bei Verwendung von reaktionsfähigen Säurederivaten z.B. in Gegenwart von basischen Mitteln, wie Triniederalkylaminen, z.B. Triäthylamin, oder heterocyclischen Base, z.B. Pyridin, durchgeführt werden. Die Acylierungsreaktion kann in Abwesenheit oder in Gegenwart eines Lösungsmittels oder Lösungsmittelgemisches, unter Kühlen, bei Raumtemperatur oder unter Erwärmen, und, wenn notwendig, in einem geschlossenen Gefäss und/oder in einer Inertgas-, z.B. Stickstoffatmosphäre, durchgeführt werden. Geeignete Lösungsmittel sind z.B. gegebenenfalls substituierte, insbesondere gegebenenfalls chlorierte, aliphatische, cycloaliphatische oder aromatische Kohlenwasserstoffe, wie Benzol und Toluol, wobei man geeignete Veresterungsreagentien, wie Essigsäureanhydrid, aus auch Verdünnungsmittel verwenden kann.

Eine durch eine organische Sulfonsäure $R_1^b$-OH, z.B. Niederalkansulfonsäure, wie Methansulfonsäure, oder eine aromatische Sulfonsäure, wie p-Toluolsulfonsäure, veresterte Hydroxygruppe kann man vorzugsweise durch Behandeln einer Hydroxymethylverbindung der Formel (I) mit einem reaktionsfähigen Sulfonsäurederivat, wie einem entsprechenden Halogenid, z.B. Chlorid, wenn notwendig, in Gegenwart

eines Säure-neutralisierenden basischen Mittels, z.B. einer anorganischen oder organischen Base, z.B. in analoger Weise wie die entsprechenden Ester mit organischen Carbonsäuren, bilden.

Eine durch eine anorganische Säure $R_1^b$-OH veresterte Hydroxygruppe wird auf übliche Weise durch Behandeln einer Hydroxymethylverbindung der Formel (I), worin die 3-Carboxylgruppe bevorzugt verestert ist, mit der anorganischen Säure, gegebenenfalls in Gegenwart eines Kondensationsmittels, oder mit einem reaktionsfähigen Derivat, z.B. einem Anhydrid oder Halogenid, davon, oder auch durch Alkylierung der anorganischen Säure oder eines Salzes davon mit einem reaktionsfähigen Derivat der Hydroxymethylverbindung der Formel (I), z.B. worin $R_1$ Halogen, z.B. Chlor, Brom oder Jod, ist, gebildet. Beispielsweise kann in einer Verbindung der Formel (I), worin $R_1$ Hydroxy ist und $R_2$ bevorzugt in geschützter Form vorliegt, die Hydroxygruppe $R_1$ durch Behandeln mit Schwefelsäure, Chlorsulfonsäure, dem Addukt aus Schwefeltrioxid und einem tert.Amin, z.B. Triäthylamin, oder mit Amidosulfonsäure und gegebenenfalls nachträglicher Neutralisation, z.B. mit Natriumbicarbonat, in eine Sulfonyloxygruppe $HOSO_2$-O- bzw. ein Salz davon übergeführt werden, oder durch Behandeln mit konzentrierter Phosphorsäure, gegebenenfalls in Gegenwart eines Kondensationsmittels, z.B. Carbodiimid oder Trichloracetonitril, mit einem Salz davon, z.B. einem Di-tertiärem-ammoniumphosphat, wie Ditriäthylammoniumphosphat, oder mit einem Anhydrid der Phosphorsäure, z.B. mit Polyphosphorsäure, Metaphosphorsäure oder Phosphor-(V)-oxid, oder einem gemischten Anhydrid, z.B. Phosphoroxychlorid, und gegebenenfalls nachträglicher Hydrolyse und/oder Neutralisation, in eine Gruppe $H_2PO_3$-O- oder ein Salz davon umgewandelt werden.

Veresterung einer freien Carboxylgruppe: Die Umwandlung von freiem Carboxyl, in erster Linie einer entsprechenden Gruppe $R_2$, in einer Verbindung der Formel (I) in verestertes Carboxyl, insbesondere in eine veresterte Carboxylgruppe, die unter physiologischen Bedingungen spaltbar ist, erfolgt nach an sich bekannten Veresterungsmethoden, beispielsweise indem man eine Verbindung der Formel(I) worin andere,

gegebenenfalls vorhandene funktionelle Gruppen gegebenenfalls in geschützter Form vorliegen, oder ein reaktionsfähiges funktionelles Carboxyderivat, inkl. ein Salz davon, mit einem entsprechenden Alkohol oder einem reaktionsfähigen funktionellen Derivat davon, umsetzt.

Die Veresterung von freiem Carboxyl mit dem gewünschten Alkohol wird in Gegenwart eines geeigneten Kondensationsmittels durchgeführt. Uebliche Kondensationsmittel sind z.B. Carbodiimide, beispielsweise N,N'-Diäthyl-, N,N'-Dipropyl-, N,N'-Dicyclohexyl- oder N-Aethyl-N'-(3-dimethylaminopropyl)-carbodiimid, geeignete Carbonylverbindungen, beispielsweise Carbonyldiimidazol, oder 1,2-Oxazoliumverbindungen, z.B 2-Aethyl-5-phenyl-1,2-oxazolium-3'-sulfonat und 2-tert.-Butyl-5-methyl-isoxazolium-perchlorat, oder eine geeignete Acylaminoverbindung, z.B. 2-Aethoxy-1-äthoxycarbonyl-1,2-dihydrochinolin. Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran und, wenn notwendig, unter Kühlen oder Erwärmen und/oder in einer Inertgasatmosphäre durchgeführt.

Geeignete reaktionsfähige funktionelle Derivate der zu veresternden Carboxylverbindungen der Formel I sind z.B. Anhydride, insbesondere gemischte Anhydride, und aktivierte Ester.

Gemischte Anhydride sind z.B. diejenigen mit anorganischen Säuren, wie Halogenwasserstoffsäuren, d.h. die entsprechenden Säurehalogenide, z.B. -chloride oder -bromide, ferner Stickstoffwasserstoffsäure, d.h. die entsprechenden Säureazide, sowie phosphorhaltigen Säuren, z.B. Phosphorsäure, Diäthylphosphorsäure oder phosphoriger Säure, oder schwefelhaltigen Säuren, z.B. Schwefelsäure, oder Cyanwasserstoffsäure. Gemischte Anhydride sind weiter z.B. diejenigen mit organischen Carbonsäuren, wie mit gegebenenfalls, z.B. durch Halogen, wie Fluro oder Chlor, substituierten Niederalkancarbonsäuren, z.B. Pivalinsäure oder Trichloressigsäure, oder mit Halbestern,

insbesondere Niederalkylhalbestern der Kohlensäure, wie Aethyl- oder Isobutylhalbestern der Kohlensäure, oder mit organischen, insbesondere aliphatischen oder aromatischen, Sulfonsäure, z.B. p-Toluolsulfonsäure.

Zur Reaktion mit dem Alkohol geeignete aktivierte Ester sind z.B. Ester mit vinylogen Alkoholen (d.h. Enolen), wie vinylogen Niederalkenolen, oder Iminomethylesterhalogeniden, wie Dimethyliminomethylesterchlorid (hergestellt aus der Carbonsäure und Dimethylchlormethyliden-iminium-chlorid der Formel $[(CH_3)_2N{=}CHCl]^{\oplus}Cl^{\ominus}$), oder Arylester, wie Pentachlorphenyl-, 4-Nitrophenyl- oder 2,3-Dinitrophenylester, heteroaromatische Ester, wie Benztriazol-, c.B. 1-Benztriazolester, oder Diacyliminoester, wie Succinylimino- oder Phthalyliminoester.

Die Acylierung mit einem solchen Säurederivat, wie einem Anhydrid, insbesondere mit einem Säurehalogenid wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt.

Ein reaktionsfähiges funktionelles Derivat des veresternden Alkohols ist in erster Linie ein entsprechender Ester, vorzugsweise mit einer starken anorganischen oder organischen Säure und stellt insbesondere ein entsprechendes Halogenid, z.B. Chlorid, Bromid oder Jodid, oder eine entsprechende Niederalkyl- oder Aryl-, wie Methyl-oder 4-Methylphenylsulfonyloxyverbindung dar.

Ein solcher reaktionsfähiger Ester eines Alkohols kann mit der freien Carboxylverbindung der Formel I oder einem Salz, wie einen Alkalimetall- oder Ammoniumsalz davon umgesetzt werden, wobei man bei Verwendung der freien Säure vorzugsweise in Gegenwart eines säurebindenden Mittels arbeitet.

Die obigen Veresterungsreaktionen werden in einem inerten, üblicherweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder Mischungen davon, wenn notwendig, unter Kühlen oder Erwärmen, z.B. in einem Temperaturbereich von etwa bei -40°C bis etwa +100°C, vorzugsweise bei etwa -10°C bis etwa +40°C, und/oder in einer Inertgas-, z.B. Stickstoffatmosphäre.

Ferner kann das Säurederivat, wenn erwünscht, in situ gebildet werden. So erhält man z.B. ein gemischtes Anhydrid durch Behandeln der Carbonsäureverbindung mit entsprechend geschützten funktionellen Gruppen oder eines geeigneten Salzes davon, wie eines Ammoniumsalzes, z.B. mit einem organischem Amin, wie Piperidin oder 4-Methylmorpholin, oder eines Metall-, z.B. Alkalimetallsalzes mit einem geeigneten Säurederivat, wie dem entsprechenden Säurehalogenid einer gegebenenfalls substituierten Niederalkancarbonsäure, z.B. Trichloracetylchlorid, mit einem Halbester eines Kohlensäure-halbhalogenids, z.B. Chlorameisensäureäthylester oder -isobutylester, oder mit einem Halogenid einer Diniederalkylphosphorsäure, z.B. Diäthylphosphorbromidat, und verwendet das so erhältliche gemischte Anhydrid ohne Isolierung.

Abspaltung von Schutzgruppen: In einer erhaltenen Verbindung der Formel I, worin eine oder mehrere funktionelle Gruppen geschützt sind, können diese, z.B. geschützte Carboxyl-, Amino-, Hydroxy- und/oder Sulfogruppen, in an sich bekannter Weise, mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall, z.B. Natriumdithionit, oder mit einem reduzierenden Metall, z.B. Zink, oder Metallsalz, wie einem Chrom-II-salz, z.B. Chrom-IIchlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxyl überführt. Durch Behandeln mit einem reduzierenden Metall oder Metallsalz, wie oben beschrieben, kann man auch 2-Halogen-niederalkoxycarbonyl (gegebenenfalls nach Umwandlung einer 2-Brom-niederalkoxycarbonylgruppe) in eine entsprechende 2-Jod-niederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumjodid, gespalten werden kann. Substituiertes 2-Silyläthoxycarbonyl kann auch durch Behandeln mit einem, das Fluoridanion liefernden Salz der Fluorwasserstoffsäure, wie einem Alkalimetallfluorid, z.B. Natrium- oder Kaliumfluorid, in Anwesenheit eines macrocyclischen Polyäthers ("Kronenäther"), oder mit einem Fluorid einer organischen quaternären Base, wie Tetra-niederalkylammoniumfluorid oder Triniederalkyl-aryl-ammoniumfluorid, z.B. Tetraäthylammoniumfluorid oder Tetrabutylammoniumfluorid, in Gegenwart eines aprotischen Lösungsmittels, wie Dimethylsulfoxid oder N,N-Dimethylacetamid, in freies Carboxyl übergeführt werden. Mit einer organischen Silyl- oder Stannylgruppe, wie Triniederalkylsilyl oder -stannyl, z.B. Trimethylsilyl, verestertes Carboxyl kann in üblicher Weise solvolytisch, z.B. durch Behandeln mit Wasser, einem Alkohol oder Säure freigesetzt werden.

Eine geschützte Aminogruppe, z.B. Am, setzt man in an sich bekannter und je nach Art der Schutzgruppen in verschiedenartiger Weise, vorzugsweise mittels Solvolyse oder Reduktion, frei. 2-Halogen-niederalkoxycarbonylamino (gegebenenfalls nach Umwandlung einer 2-Brom-niederalkoxycarbonylaminogruppe in eine 2-Jod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z.B. Natriumdithionit, gespalten werden. Gegebenenfalls

substituiertes Diphenylmethoxycarbonylamino, tert.-Niederalkoxycarbonylamino oder 2-trisubstituiertes Silyläthoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino, Formylamino oder 2-Acyl-niederalk-1-en-yl-amino, z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silyl- oder Stannylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsproduktes freigesetzt werden. Eine durch 2-substituiertes Silyläthoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxylgruppe angegeben, in die freie Aminogruppe übergeführt werden. Eine Phosphor-, Phosphon- oder Phosphin-amidogruppe kann z.B. durch Behandeln mit einer Phosphorhaltigen Säure, wie einer Phosphor-, Phosphon- oder Phosphinsäure, z.B. Orthophosphorsäure oder Polyphosphorsäure, einem sauren Ester, z.B. Monomethyl-, Monoäthyl-, Dimethyl- oder Diäthylphosphat oder Monomethylphosphonsäure, oder einem Anhydrid davon, wie Phosphorpentoxid, in die freie Aminogruppe übergeführt werden.

Ein in Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch

durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20 bis 25°C, oder auch unter Kühlen oder Erwärmen, durchgeführt.

Eine durch eine geeignete Acylgruppe, eine organische Silyl- oder Stannylgruppe oder durch gegebenenfalls substituiertes 1-Phenylniederalkyl geschützte Hydroxygruppe wird wie eine entsprechend geschützte Aminogruppe freigesetzt. Eine durch 2,2-Dichloracetyl geschützte Hydroxygruppe wird z.B. durch basische Hydrolyse, eine durch tert.-Niederalkyl oder durch einen 2-oxa- oder 2-thiaaliphatischen oder -cycloaliphatischen Kohlenwasserstoffrest verätherte Hydroxygruppe durch Acidolyse, z.B. durch Behandeln mit einer Mineralsäure oder einer starken Carbonsäure, z.B. Trifluoressigsäure, freigesetzt. Eine 2-Halogenniederalkylgruppe wird reduktiv abgespalten.

Eine geschützte, insbesondere veresterte Sulfogruppe wird analog einer geschützten Carboxylgruppe freigesetzt.

Die beschriebenen Spaltungsreaktionen werden unter an sich bekannten Bedingungen durchgeführt, wenn notwendig unter Kühlen oder Erwärmen, in einem geschlossenen Gefäss und/oder in einer Inertgas-, z.B. Stickstoffatmosphäre.

Die an sich bekannten reduktiven Spaltungsmethoden von 2-Halogenniederalkoxycarbonyl- und 2-Halogenniederalkoxygruppen können in vorteilhafter Weise unter besonders milden Bedingungen in Anwesenheit einer katalytischen Menge eines Uebergangsmetall-Komplexes einer Corrin- oder Porphin-Verbindung durchgeführt werden, wobei auch üblicherweise schwer spaltbare Gruppen, z.B. 2-Chloräthoxycarbonyl- und 2-Chloräthoxygruppen, gespalten werden können.

Uebergangsmetall-Komplexe von Corrin- und Porphin-Verbindungen, die man als Katalysatoren der reduktiven Abspaltung einsetzt, leiten sich von solchen Uebergangsmetallen ab, die in mehr als einer Wertigkeitsstufe auftreten, wie Kupfer, Palladium, Rhodium, und vor allem Kobalt. Bevorzugt als Katalysator sind Modellverbindungen zur Synthese von Vitamin $B_{12}$, z.B. Derivate, welche sich vom 2,2,3,3,/,7,8,8,12,12,13,13,17,17,18,18-Hexadecamethyl-10,20-diazahexahydroporphin-Kobalt(III)-Kation ableiten, und insbesondere Vitamine der $B_{12}$-Gruppe, darunter vor allem Cyanocobalamin, Aquocobalamin und Hydroxycobalamin, sowie ihre Derivate und Abbauprodukte, wie Cobyrinsäure und ihre Ester, Corphyrinsäure, Corphinsäure, Cobaminsäure und Cobamid. Die Menge des Katalysators beträgt im Normalfall etwa 0,1 bis etwa 0,001, vorzugsweise etwa 0,03 bis etwa 0,003 Moläquivalent, bezogen auf die zu reduzierende Komponente, bei einer günstigen Versuchsanordnung, z.B. bei einer optimalisierten Elektroreduktion, kann man aber die unterste Grenze noch weit unterschreiten.

Die Reduktion wird in an sich bekannter Weise durch konventionelle Methoden in Anwesenheit einer Protonenquelle durchgeführt. Als diese können beliebige anorganische und organische Säuren sowie gebräuchliche protische Lösungsmittel dienen; Säuren werden mit Vorteil in einer gepufferten Form oder als Salze eingesetzt, wobei der pH-Wert vorzugsweise zwischen etwa 4,0 bis 9,5, insbesondere zwischen etwa 5,5 bis 7,5 und vor allem in der Nähe des Neutralpunktes liegt.

Als eine der schonendsten Reduktionsmethoden empfiehlt sich die kathodische Elektroreduktion unter konventionellen Bedingungen, wie an der Quecksilber- oder einer anderen für die Elektroreduktion allgemein verwendbaren Kathode, wie Kohle-Kathode, in Anwesenheit

von üblichen Hilfselektrolyten, wie Ammonium- und/oder Alkalimetallsalzen, insbesondere Lithiumsalzen, starker anorganischer Säuren, wie insbesondere Halogeniden und Perchloraten, vorzugsweise solchen, die sich durch eine gute Löslichkeit im verwendeten wässrig-organischen Milieu auszeichnen. Als Lösungsmittel dienen, neben Wasser, übliche mit Wasser mischbare organische Lösungsmittel, z.B. Niederalkanole, wie Methanol, Aethanol und Isopropylalkohol, oder Dimethylformamid, und ähnliche niederaliphatische Amide, ferner auch Nitrile, wie Acetonitril, Aether, wie Diäthyläther, 1.2-Dimethoxy- und 1,2-Diäthoxy-äthan und cyclische Aether vom Typ Tetrahydrofuran, Ketone, wie Aceton, Carbonate,wie Dimethyl- und Diäthylcarbonat, sowie Sulfoxide, wie insbesondere Dimethylsulfoxid. Die Temperatur der Elektroreduktion kann in einem breiten Bereich variiert werden, d.h. von etwa -20°C bis zur Siedetemperatur des Reaktionsgemisches, vorzugsweise liegt sie bei Raumtemperatur oder darunter.

Als eine weitere geeignete Variante der reduktiven Spaltung kommt die Reduktion mit einem Metall, vor allem mit Zink oder auch mit Magnesium, in Betracht. Dabei kann man unter Umständen die üblichen energischen Bedingungen, z.B. etwa 90%ige wässrige Essigsäure als Milieu und/oder erhöhte Temperatur bis zum Siedepunkt des Gemisches bei entsprechend verkürzter Reaktionszeit anwenden. Vorzugsweise arbeitet man aber unter möglichst schonenden Bedingungen, z.B. im oben erwähnten bevorzugten pH-Bereich in der Nähe des Neutralpunktes und bei Temperaturen von etwa 0° bis etwa 40°C, vorzugsweise etwa 10-25°C; üblicherweise arbeitet man mit einem etwa zehnfachen molaren Ueberschuss am Metall, vorzugsweise Zink in Form von Zinkstaub, den man mit Vorteil noch unmittelbar vor der Reaktion in konventioneller Weise, z.B. durch Verrühren mit verdünnter Salzsäure und sorgfältiges Neutralwaschen, aktiviert. Zum Erzielen der gewünschten Pufferwirkung können mit Vorteil Ammoniumsalze von Halogenwasserstoffsäuren dienen, insbesondere von denjenigen, die in der jeweiligen abzuspaltenden Gruppe der Formel I vorkommen. Die Reduktion erfolgt

in den oben erwähnten organischen Lösungsmitteln oder deren Gemischen; protische Lösungsmittel sind bevorzugt. Auch andere herkömmliche Varianten des Reduktionsverfahrens sind anwendbar: als Beispiel sei die Reduktion mit Metall-Kationen in tiefen Wertigkeitsstufen, z.B. mit Chrom(II)-Salzen, wie Chrom(II)-chlorid oder -acetat, ferner auch die Reduktion mit komplexen Metallhydriden, z.B. mit Natriumborhydrid und verwandten Niederalkoxy-natriumborhydriden und Niederalkoxylithiumborhydriden, sowie die katalytische Hydrierung, z.B. an einem Palladium-Katalysator, erwähnt.

Bevorzugt werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium/Kohle/Katalysator.

Salzbildung: Salze von Verbindungen der Formel I mit salzbildenden Gruppen können in an sich bekannter Weise hergestellt werden. So kann man Salze von Verbindungen der Formel I mit sauren Gruppen z.B. durch Behandeln mit Metallverbindungen, wie Alkalimetallsalzen von geeigneten organischen Carbonsäuren, z.B. dem Natriumsalz der α-Aethyl-capronsäure, oder mit anorganischen Alkali- oder Erdalkalimetallsalzen, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel I, welche z.B. eine freie Carboxylgruppe enthalten, können z.B. durch Neutralisieren von Salzen, wie Säureadditionssalzen, auf

den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Das Sulfoxid der Formel (IIa), worin der Index n den Wert 1 hat, kann in der 1R- oder 1S-Konfiguration oder als Gemisch der beiden Isomeren eingesetzt werden.

Ebenso kann in einem Ausgangsmaterial der Formeln (IIa-c) die 6-Stellung die R-, S- oder die R,S-Konfiguration haben. Bei der Oxidation bleibt die Konfiguration am 6-Kohlenstoffatom erhalten. Falls ein Gemisch der 6R- und 6S-Verbindungen der Formel (IIa) eingesetzt wird, erhält man ein Gemisch der 6R- und 6S-Verbindungen der Formel (I). Falls ein Gemisch der 6R- und 6S-Verbindungen der Formeln (IIb) und (IIc) eingesetzt wird, erhält man bevorzugt die 6R-Verbindung der Formel (I).

Gemische von Isomeren können in an sich bekannter Weise, z.B. durch fraktionierte Kristallisation, Chromatographie, etc. in die einzelnen Isomeren aufgetrennt werden.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Ausgangsstoffe: Die im Verfahren zur Herstellung der Verbindungen der vorliegenden Erfindung verwendeten Ausgangsstoffe der Formeln (IIa) und (IIc) sind neu. Sie können in an sich bekanter Weise hergestellt werden. Zusammen mit den Verfahren zu ihrer Herstellung bilden sie ebenfalls einen Gegenstand der vorliegenden Erfindung. Verbindungen der Formeln (IIa) und (IIc), worin $R_2$ Carboxyl oder unter physiologischen Bedingungen spaltbares, verestertes Carboxyl ist, und die pharmzeutisch annehmbaren Salze von solchen Verbindungen, die eine salzbildende Gruppe aufweisen, haben ähnliche pharmazeutische Eigenschaften wie die Verbindungen der Formel (I).

Die Verbindungen der Formel (IIa) können beispielsweise hergestellt werden, indem man

a') in eine Verbindung der Formel

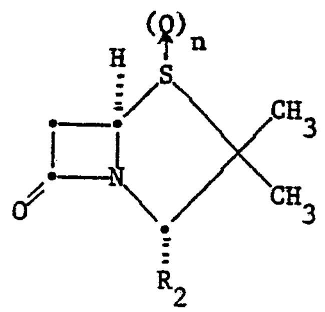

(III),

worin der Index n den Wert 0 oder 1 hat und $R_2$ eine veresterte Carboxylgruppe darstellt, die Gruppe $R_1$-$CH_2$- einführt, oder

b') in einer Verbindung der Formel

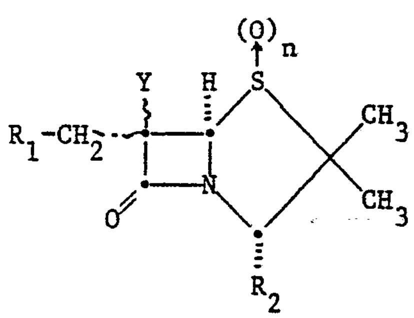

(IV),

worin $R_1$, $R_2$ und der Index n die unter Formel(IIa) genannten Bedeutungen haben und Y eine in ein Wasserstoffatom überführbare Gruppe darstellt, die Gruppe Y in ein Wasserstoffatom überführt, und, wenn erwünscht oder notwendig, in einer erhältlichen Verbindung eine Gruppe $R_1$ in eine andere Gruppe $R_1$ überführt, und/oder eine Gruppe $R_2$ in eine andere Gruppe $R_2$ überführt, und/oder ein erhältliches 1-Sulfid in ein 1-Sulfoxid oder ein erhältliches 1-Sulfoxid in ein 1-Sulfid überführt, und/oder ein erhältliches Salz in die freie Verbindung oder in ein anderes Salz überführt, und/oder eine erhältliche freie Verbindung mit einer salzbildenden Gruppe in ein Salz überführt, und/oder ein erhältliches Gemisch von isomeren Verbindungen der Formel (I) in die einzelnen Isomeren auftrennt.

Die Einführung einer Gruppe $R_1$-$CH_2$- und der Austausch Y gegen Wasserstoff erfolgt analog den unter b) und c) beschriebenen Verfahren.

Die gemäss den Verfahren (a') und (b') erhältlichen Verbindungen der Formel (IIa) können durch Nachoperationen in andere Verbindungen der Formel (IIa) übergeführt werden, wobei zur Umwandlung von $R_1$ in ein anderes $R_1$, von $R_2$ in ein anderes $R_2$, für die Salzherstellung und die Herstellung der freien Verbindungen aus den Salzen, sowie die Auftrennung der Isomerengemische in die einzelnen Isomeren die gleichen Verfahren angewendet werden können, wie für die entsprechenden Nachoperationen bei den Verfahren zur Herstellung von Verbindungen der Formel (I) angegeben ist.

Auch die Herstellung der 1-Sulfoxide der Verbindungen der Formel (IIa), worin der Index n den Wert 1 hat, aus den 1-Sulfiden der Formel (IIa), worin der Index n den Wert 0 hat, kann analog der Oxidation von Verbindungen der Formel (IIa) zu den Sulfonen der Formel (I) erfolgen, wobei die Menge des Oxidationsmittels entsprechend zu reduzieren ist, wenn eine Ueberoxidation vermieden werden soll.

Die Reduktion von 1-Sulfoxiden der Formel (IIa) zu den 1-Sulfiden erfolgt mittels der in der Penicillin- und Cephalosporinchemie bekannten Methoden, beispielsweise durch Behandeln mit Phosphortrichlorid oder -bromid.

Die Verbindungen der Formel (IIb) sind bekannt oder können auf an sich bekante Weise hergestellt werden.

Die Verbindungen der Formel (IIc) können aus Verbindungen der Formel (IV) durch Oxidation analog dem Verfahren a) hergestellt werden.

Die Ausgangsverbindungen der Formel (III) und ihre 6-Halogenderivate sind bekannt oder können auf an sich bekannte Weise hergestellt werden.

Die Ausgangsverbindungen der Formel (IV) können auf an sich bekannte Weise hergestellt werden, beispielsweise, indem man in eine Verbindung der Formel

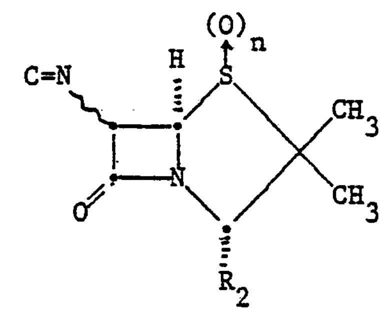

(V)

oder

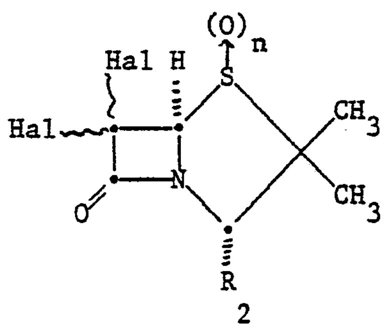

(VI)

worin der Index n den Wert 0 oder 1 hat, $R_2$ eine veresterte Carboxylgruppe, z.B. Chlor, Jod oder insbesondere Brom, darstellt, die Gruppe $R_1-CH_2-$ analog einem der unter b) genannten Verfahren zur Herstellung von Verbindungen der Formel(IIa) einführt. Falls eine Verbindung der Formel (V) als Ausgangsmaterial benutzt wird, stellt man das Anion bevorzugt mit Kaliumcarbonat in Dimethylformamid her, z.B. gemäss P.H. Bentley und J.P. Clayton, J.C.S. Chem. Comm. 1974, S. 278.

Die Verbindungen der Formeln (V) und (VI) sind bekannt oder auf an sich bekannte Weise herstellbar.

Die Verbindungen der vorliegenden Erfindung zeichnen sich durch eine hervorragende β-Lactamase-inhibierende Wirkung aus, die sich beispielsweise in vitro durch die Inhibierung von β-Lactamasen aus Staphylococcus aureus, Enterobacter cloacae, Pseudomonaden, Morganella morganii, Escherichia coli, Haemophilus influenzae und Bacteroides fragilis nachweisen lässt. Insbesondere die bevorzugten Verbindungen zeichnen sich durch eine $I_{50}$ (Konzentration der Testsubstanz die 50% der β-Lactamase hemmt) von etwa 1.2 bis 0.026 µM aus.

Aufgrund dieser β-Lactamase-inhibierenden Wirkung erhöhen die Verbindungen der vorliegenden Erfindung die Wirkung von β-Lactamantibiotika <u>in vitro</u> und <u>in vivo</u>, wie durch Vergleich der antibiotischen Wirkungen von Verbindungen der vorliegenden Erfindung und von β-Lactamantibiotika allein und in Kombination miteinander festgestellt werden kann. Beispielsweise liegen <u>in vitro</u> die MIC-Werte von 1:1 Kombinationen der erfindungsgemässen Verbindung der Formel (I), worin $R_1$ Hydroxy und $R_2$ Carboxy bedeuten, oder des Natriumsalzes davon mit Ampicillin, Cefsulodin (CGP7174/E) oder Cefotiam (CGP14221/E) gegen eine Reihe der obengenannten β-Lactamase produzierenden Mikroorganismen signifikant niedriger als die MIC-Werte der Einzelverbindungen. <u>In vivo</u>, beispielsweise bei subcutaner Applikation an Mäusen, die

mit β-Lactamase produzierenden Mikroorganismen infiziert wurden, z.B. mit Escherichia coli 205 $R^+$ TEM oder Morganella morganii 2359, wurde eine signifikante Erniedrigung der $ED_{50}$-Werte gegenüber denjenigen der Einzelkomponenten festgestellt. Einige Werte sind in der folgenden Tabelle zusammengestellt:

| infizierender Mikroorganismus | s.c. $ED_{50}$ (mg/kg, Maus) | | |
|---|---|---|---|
| | A | B | A + B (1:1) |
| Esch.coli: 205 RT | 100 | 70 | 14 |
| Morganella morg. 2359 | 100 | 75 | 18 |

A = (3R, 5R, 6R)-2,2-Dimethyl-6-hydroxymethylpenam-3-carbonsäure-1,1-dioxid-natriumsalz

B = Cefotiam.

Die β-Lactamase-hemmenden Verbindungen der vorliegenden Erfindung, inklusive ihre pharmazeutisch anwendbaren Salze, sind demnach wertvoll in der Behandlung von Infektionen an Mensch und Tier, die durch β-Lactamase-produzierende Mikroorganismen hervorgerufen werden und bei deren Bekämpfung β-Lactamase-empfindliche Antibiotika eingesetzt werden. Die aktiven Verbindungen der vorliegenden Erfindung können entweder vor oder insbesondere gleichzeitig mit dem eigentlich wirksamen Antibiotikum verabreicht werden. Die Dosis an erfindungsgemässen β-Lactamaseinhibitoren beträgt etwa 1/10 bis etwa das zehnfache, insbesondere etwa 1/5 bis etwa das fünffache, der Menge an einzusetzendem Antibiotikum.

Die pharmakologisch verwendbaren Verbindungen der vorliegenden Erfindung können zur Herstellung von pharmazeutischen Präparaten verwendet werden, welche eine wirksame Menge der erfindungsgemässen Aktivsubstanz entweder allein oder im Gemisch mit

anorganischen oder organischen, festen oder flüssigen, pharmazeutisch verwendbaren Trägerstoffen und gegebenenfalls ein β-Lactamantibiotikum enthalten, die sich zur oralen oder zur parenteralen, d.h. intramuskulären, subkutanen oder intraperitonealen,Verabreichung eignen.

Die pharmakologisch verwendbaren Verbindungen der vorliegenden Erfindung können zur Herstellung von pharmazeutischen Präparaten verwendet werden, welche eine wirksame Menge der erfindungsgemässen Aktivsubstanz entweder allein oder im Gemisch mit anorganischen oder organischen, festen oder flüssigen, pharmazeutisch verwendbaren Trägerstoffen und gegebenenfalls einem β-Lactamantibiotikum enthalten, die sich zur oralen oder zur parenteralen, d.h. intramuskulären, subcutanen oder intraperitonealen, Verabreichung eignen.

Zur Herstellung von pharmazeutischen Präparaten, die neben einer β-Lactamase-inhibierenden Verbindung der vorliegenden Erfindung, noch ein β-Lactamantibiotikum enthalten, können irgendwelche die β-Lactamstruktur enthaltende Antibiotika benutzt werden, wobei Penicillin-, Cephalosporin-, und ferner die Penem-Antibiotika, die gegen β-Lactamasen instabil sind, bevorzugt sind.

Geeignete Penicilline und Cephalosporine können den zusammenfassenden Publikationen von E.H. Flynn, Cephalosporins and Penicillins, Academic Press, New York und London, 1972, P.G. Sammes, Chem. Rev., 1976, Vol. 76, No. 1, Seite 113-155, J.Cs. Jászberényi and T.E. Gunda, Progr. Med. Chem., Vol. 12, 1975, Seite 395-477, J. Elks, Recent Advances in the Chemistry of β-Lactam-Antibiotics, The Chemical Society, Burlington House, London W1V OBN, 1977, dem Merck Index, 9th Ed., Merck & Co., Inc., Rahway, N.J., USA, 1976, und der Encyclopaedia of Antibiotics, sec. Ed., John Wiley & Sons (1978) entnommen werden.

Geeignete Penicilline sind beispielsweise Penicillin V (z.B. als Kaliumsalz), Benzylpenicillin (z.B. als Kalium- oder Natriumsalz), Propicillin (z.B. als Kaliumsalz), Epicillin, Cyclacillin, Azidocillin oder Penicilline aus der Gruppe der gegebenenfalls substituierten α-Aminobenzylpenicilline, z.B. Ampicillin, Amoxicillin, Apalcillin, Pirbenicillin, Hetacillin, Metampicillin oder 4-Acetoxyampicillin.

Geeignete Penicilline sind auch solche aus den Gruppen der Carboxypenicilline, Sulfopenicilline, Ureidopenicilline, Acylureidopenicilline und Methylenaminopenicilline.

Unter die Gruppen der Carboxy- und Sulfopenicilline fallen beispielsweise Carbenicillin (Natriumsalz), Carfecillin, Indanylcarbenicillin, Ticarcillin (Dinatriumsalz), und Sulfocillin (Sulbenicillin; z.B. als Dinatriumsalz).

Für die genannten pharmazeutischen Mischungen geeignete Ureidopenicilline und Acylureidopenicilline sind beispielsweise Azlocillin, Mezlocillin und Furazlocillin und die 6β-[D-2(4-A-2,3-Dioxo-1-piperazinocarboxamido)phenylacetamido]penicillansäure, worin A Wasserstoff oder ein Substituent, insbesondere Niederalkyl, z.B. Methyl oder Aethyl, ist. Besonders hervorzuheben ist das Piperacillin. Die Ureidopenicilline und Acylureidopenicilline werden bevorzugt als Salze, insbesondere als Natriumsalze, eingesetzt.

Geeignete Methylenaminopenicilline sind beispielsweise 6β-(1-Azacyclylmethylenamino)-penicillansäuren, insbesondere Mecillinam und deren Salze, wie Natriumsalze, sowie Pivmecillinam.

Geeignete Cephalosporine sind beispielsweise Cefaclor, Cefadroxil, Cefamandol, Cefamandol-nafat, Cefaparol, Cefatrizin, Cefazolin, Cefonicid, Cefoperazon, Ceforanid, Cefotaxime, Cefotiam (als Dihydrochlorid), Cefroxadin, Cefsulodin, Ceftezol, Cephalexin, Cephaloglycin, Cephaloridin, Cephalothin, Cephapyrin (Natriumsalz), Cepha-

drin, Cefatriaxon, 7β-[2-(2-Amino-thiazol-4-yl)-2-(2-carboxy-prop-2-yloxyimino)acetamido]-2-pyridinio-3-cephem-4-carboxylat (GR 20263), 7β-[2-(2-Aminothiazol-4-yl)-2-methoxyimino-acetamido]-3-(1-methyl-tetrazol-5-ylthiomethyl)-3-cephem-4-carbonsäure-natriumsalz (SCE 1365), 7β-[2-(2-Aminothiazol-4-yl)-2-syn-methoxyimino)acetamido]-3-acetoxymethyl-3-cephem-4-carbonsäure (CGP 17845), 7β-[2-(2-Aminothiazol-4-yl)-2-syn-methoxyimino)acetamido]-3-acetoxymethyl-3-cephem-4-carbonsäure-pivaloyloxymethylester (gegebenenfalls als Hydrochlorid (CGP 18658/A)), 7β-{(2R)-2-[4-((2R)-2-Amino-2-carboxyäthoxycarbonylamino)phenyl]-2-hydroxyacetylamino}-3-[(1-methyl-1H-tetrazol-5-yl)-thiomethyl]-3-cephem-4-carbonsäure (Natriumsalz; CGP 17520), sowie auch 1-Oxacephalosporine, z B. 7β-[2-(p-Hydroxyphenyl)-2-carboxy-acetamido]-3-[(1-methyl-1H-tetrazol-5-yl)thiomethyl]-1-dethia-1-oxa-3-cephem-4-carbonsäure (Dinatriumsalz; LY 127935 oder S 6059).

Geeignete Penem-Antibiotika sind aus der europäischen Patentanmeldung 3960 bekannt und umfassen beispielsweise 6-$R_1$-2-$R_2$-Penem-3-carbonsäuren, worin $R_1$ Niederalkyl oder Hydroxyniederalkyl und $R_2$ Wasserstoff, gegebenenfalls substituiertes Niederalkyl oder Niederalkylthio bedeutet, z.B. (5R,6R)-6-Hydroxymethyl-penem-3-carbonsäure und (5R,6R)-6-[(1R)-1-Hydroxyäthyl]-penem-3-carbonsäure und ihre Salze, z.B. die Natriumsalze.

An Stelle der freien Carbonsäuren der genannten β-Lactamantibiotika oder ihren Salzen können auch die üblichen physiologisch spaltbaren Ester, beispielsweise die Pivaloyloxymethylester, Acetoxymethylester, 1-Aethoxycarbonyloxymethyl- oder-äthylester oder die Phthalidylester, an Stelle der genannten freien Verbindungen auch deren pharmazeutisch annehmbaren Salze, inkl. die inneren Salze und Hydrate, und an Stelle der genannten Salze auch deren freie Verbindungen in die Mischung eingesetzt werden. Die Wahl richtet sich nach der Applikationsart, dem gewünschten pH-Wert und/oder der gewünschten Konzentration einer zu injizierenden Lösung.

Das Verhältnis von β-Lactamase-inhibierenden Verbindungen der vorliegenden Erfindung zu dem eigentlich wirksamen Antibiotikum kann in den erfindungsgemässen pharmazeutischen Präparaten in weiten Grenzen schwanken und liegt etwa zwischen 1:10 bis 10:1, bevorzugt 1:5 bis 5:1, und ist beispielsweise 3:1, 2:1, 1:1, 1:2, 1:3, 1:4, 1:5 oder 1:6. Die Penicillin- und Cephalosporinantibiotika werden entweder in der sonst üblichen Menge in den Präparaten verarbeitet, wobei eine längere Wirkungsdauer erzielt werden kann, oder auch in geringerer als üblicher Menge, wobei die Menge aber so bemessen sein soll, dass die antibiotische Wirksamkeit etwa gleich ist, wie wenn das Präparat ohne Zusatz des β-Lactamasehemmers verabreicht wird.

Pharmazeutische Präparate oder Kombinationspräparate, die eine β-Lactamase-inhibierende Verbindung der vorliegenden Erfindung, gegebenenfalls zusammen mit einem β-Lactamantibiotikum enthalten, können auf die gleiche Weise hergestellt werden, wie für die β-Lactamantibiotika allein bekannt ist. In den Kombinationspräparaten erfolgt die Zugabe der erfindungsgemässen β-Lactamase-inhibierenden Verbindungen innerhalb der angegebenen Mischungsverhältnisse. In den Kombinationspräparaten können die beiden Wirksubstanzen entweder homogen gemischt oder nebeneinander in getrennten Kompartments vorliegen.

Vorzugsweise verwendet man die pharmakologisch wirksamen Verbindungen der vorliegenden Erfindung in Form von parenteral, z.B. intramuskulär oder intravenös, verabreichbaren Präparaten oder von Infusionslösungen. Solche Lösungen sind vorzugsweise isotonische wässrige Lösungen oder Suspensionen, wobei diese z.B. aus lyophilisierten Präparaten, welche die Wirksubstanzen allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Lösungs- oder Lyophilisierungsverfahren, hergestellt und enthalten von etwa 0,1% bis 100%, insbesondere von etwa 1% bis etwa 50%, Lyophilisate bis zu 100% der Aktivstoffe. Verbindungen der Formel I, insbesondere diejenigen, in welchen $R_2$ eine physiologisch spaltbare, veresterte Carboxylgruppe, z.B. Pivaloyloxymethoxycarbonyl, darstellt, können auch oral, z.B. als Granulat in Form von Kapseln, verabreicht werden. Die pharmazeutischen Präparate enthalten den Wirkstoff, oder gegebenenfalls die beiden Wirkstoffe, gegebenenfalls zusammen mit geeigneten Trägerstoffen, in Dosen von etwa 0,05 g bis etwa 1,0 g pro Dosiseinheit.

Je nach Art der Infektion, Zustand des infizierten Organismus und gegebenenfalls der antibiotischen Wirksamkeit des β-Lactamantibiotikums verwendet man tägliche Dosen von etwa 0,1 g bis etwa 5 g zur Behandlung von Warmblütern von etwa 70 kg Gewicht. Im allgemeinen liegt die Dosis für die Kombinationspräparate in der Grössenordnung der Dosis, in der das β-Lactamantibiotikum allein verwendet wird.

Gewisse Verbindungen der vorliegenden Erfindung verstärken die antibiotische Wirkung einiger β-Lactamantibiotika in synergistischer Weise. In solchen Fällen kann die Dosis verringert werden.

Die folgenden Beispiele dienen zur Illustration der Erfindung. Temperaturen werden in Celsiusgraden angegeben. DC bedeutet Dünnschichtchromatogramm.

Beispiel 1: (3R,5R,6R und 6S)-2,2-Dimethyl-6-hydroxymethyl-penam-3-carbonsäure-diphenylmethylester

Eine Lösung von 1,0 g (2,12 mMol) (3R,5R,6S)-2,2-Dimethyl-6-jod-penam-3-carbonsäure-diphenylmethylester in 25 ml Tetrahydrofuran wird auf -78° gekühlt und mit 1,2 ml (2,12 mMol) einer 1,8-molaren Lösung von Methylmagnesiumbromid in Diäthyläther versetzt. Nach 10 Minuten bei -78° wird unter gutem Rühren Formaldehydgas über die Lösung geleitet. Nach 1/2 Stunde zeigt eine DC-Probe vollständigen Umsatz an. Nach 40 Minuten wird die Reaktionslösung mit 0,4 ml Eisessig neutralisiert, mit Aethylacetat verdünnt und mit wässriger Natriumbicarbonatlösung und Kochsalzlösung gewaschen. Die organischen Phasen werden über Natriumsulfat getrocknet und am Rotationsverdampfer eingeengt. Der Rückstand wird an 100 g Silicagel mit dem Lösungsmittelsystem Toluol/Aethylacetat 2:1 chromatographiert und man erhält ein Gemisch der 6R- und 6S-Titelverbindung im Verhältnis 2:3.

DC: Silicagel, Toluol/Aethylacetat Rf = 0.38

IR-Spektrum ($CH_2Cl_2$): Absorptionsbanden bei 2.79; 5.63; 5.73 µ.

Beispiel 2: (1R,3R,5R,6R)-, (1S,3R,5R,6R)- und (1S,3R,5R,6S)-2,2-Dimethyl-6-hydroxymethyl-penam-3-carbonsäure-diphenylmethylester-1-oxid

Eine Lösung von 1,22 g (3 mMol) eines 2:3 Gemisches von (3R,5R,6R)- und (3R,5R,6S)-2,2-Dimethyl-6-hydroxymethylpenam-3-carbonsäure-diphenylmethylester in 30 ml Chloroform wird auf -6° gekühlt und mit 590 mg (3,09 mMol) 90%iger m-Chlorperbenzoesäure versetzt. Nach 20 Minuten wird die Reaktionslösung mit wässriger Natriumthiosulfat- und wässriger Natriumbicarbonatlösung gewaschen. Die Wasserphase wird zweimal mit Chloroform nachextrahiert. Die vereinigten Chloroformlösungen werden über Natriumsulfat getrocknet und am Rotationsverdampfer eingeengt. Der Rückstand wird an 45 g

Silicagel mit dem Laufmittelsystem Toluol/Aethylacetat 1:2 chromatographiert. Die drei Titelverbindungen wurden in der angegebenen Reihenfolge eluiert:

1. (1R,3R,5R,6R)-Isomer: DC: Silicagel, Toluol/Aethylacetat Rf = 0,23
   IR-Spektrum ($CH_2Cl_2$): Absorptionsbanden bei 2.75; 2.94; 5.59; 5.69 µ.
2. (1S,3R,5R,6R)-Isomer : DC: Silicagel, Toluol/Aethylacetat Rf = 0,21
   IR-Spektrum ($CH_2Cl_2$): Absorptionsbanden bei 2.75; 2.95; 5,57; 5,69 µ.
3. (1S,3R,5R,6S)-Isomer : DC: Silicagel Toluol/Aethylacetat Rf = 0.16; Smp. 135° bis 138°C.
   IR-Spektrum ($CH_2Cl_2$): Absorptionsbanden bei 2.76; 2.9; 5.58; 5,69 µ.

Beispiel 3: [(3R,5R,6R)-2,2-Dimethyl-6-hydroxymethyl-penam-3-carbonsäure-diphenylmethylester-1,1-dioxid

a) 3,81 g (9,22 mMol) eines Gemisches von (1R,3R,5R,6R)- und (1S,3R,5R,6R)-2,2-Dimethyl-6-hydroxymethyl-penam-4-carbonsäurediphenylmethylester-1-oxid werden in 114 ml Chloroform gelöst und mit 1,86 g (10,8 mMol) 90%iger m-Chlorperbenzoesäure bei Raumtemperatur versetzt. Nach 1 1/2-stündigem Rühren wird mit wässriger Natriumthiosulfat-, wässriger Natriumbicarbonat- und wässriger Natriumchloridlösung aufgearbeitet. Die wässrigen Phasen werden zweimal mit Chloroform nachextrahiert. Die vereinigten organischen Lösungen werden mit Natriumsulfat getrocknet und am Rotationsverdampfer eingeengt. Der Rückstand wird am Hochvakuum getrocknet und durch Chromatographie an 38 g Silicagel mit dem Lösungsmittelsystem Toluol/Aethylacetat 3:1 gereinigt. Man erhält die Titelverbindung vom Rf-Wert = 0,23 (Silicagel, Toluol/Aethylacetat).
IR-Spektrum: ($CH_2Cl_2$): Absorptionsbanden bei 2.78; 5.55; 5.68; 7.52 µ.

Die gleiche Verbindung kann auch wie folgt erhalten werden:

b) 0,5 g (1,26 mMol) (3R,5R,6R)-2,2-Dimethyl-4-hydroxymethyl-penam-4-carbonsäure-diphenylmethylester werden in 13 ml Chloroform gelöst und mit 0,5 g (2,6 mMol) 90%iger m-Chlorperbenzoesäure bei Raumtemperatur versetzt. Nach 1 1/2-stündigem Rühren wird mit wässriger Natriumthiosulfat-, wässriger Natriumbicarbonat- und wässriger Natriumchloridlösung aufgearbeitet. Die wässrigen Phasen werden zweimal mit Chloroform nachextrahiert. Die vereinigten organischen Lösungen werden mit Natriumsulfat getrocknet und am Rotationsverdampfer eingeengt. Der Rückstand wird am Hochvakuum getrocknet und durch Chromatographie an 10 g Silicagel mit dem Lösungsmittelsystem Toluol/Aethylacetat 3:1 gereinigt. Man erhält die Titelverbindung mit den gleichen analytischen Daten wie unter a) angegeben.

Beispiel 4: (3R,5R,6R)-2,2-Dimethyl-6-hydroxymethyl-penam-3-carbonsäure-1,1-dioxid-natriumsalz

a) 3,34 g (7,78 mMol) (3R,5R,6R)-2,2-Dimethyl-6-hydroxymethyl-penam-3-carbonsäure-diphenylmethylester-1,1-dioxid werden in 100 ml Tetrahydrofuran gelöst, mit 1,3 g Pd/C 10% versetzt und unter Normaldruck bei Raumtemperatur hydriert. Nach 6 Stunden Reaktionsdauer wird vom Katalysator abfiltriert und das Filtrat am Rotationsverdampfer eingeengt. Der Rückstand wird in Wasser/Aethylacetat aufgenommen und mit 653 mg (7,78 mMol) Natriumbicarbonat versetzt. Die wässrige Phase wird dreimal mit Aethylacetat extrahiert. Die organischen Phasen werden zweimal mit Wasser gewaschen. Nach Lyophilisation der vereinigten Wasserphasen wird die Titelverbindung als weisses Pulver erhalten.
IR-Spektrum (KBr): Absorptionsbanden bei 2.9; 5.63; 6.18; 7.19; 7.61 $\mu$.

Die gleiche Verbindung kann auch wie folgt erhalten werden:

b) Eine Lösung von 0,1 g (0,38 mMol) (1R,3R,5R,6R)-2,2-Dimethyl-6-hydroxymethyl-penam-3-carbonsäure- 1-oxid in 10 ml Wasser/Tetrahydrofuran 1:1 wird mit 80 mg (o,41 mMol) 90%iger m-Chlorperbenzoesäure bei Raumtemperatur versetzt. Nach 1 1/2 stündigem Rühren wird mit wässriger Natriumthiosulfatlösung versetzt. Die wässrige Phase wird mit 1-N Natronlauge auf pH = 6,8 eingestellt und lyophilisiert. Die erhaltene Titelverbindung wird durch Chromatographie auf UP $C_{12}$-Platten gereinigt (Laufmittel, $H_2O$, Rf = 0.58).

c) Die Titelverbindung wird auch analog b), ausgehend von 0,1 g (0.37 mMol) (1S,3R,5R,6R)-2,2-Dimethyl-6-hydroxymethyl-penam-3-carbonsäure-1-oxid-natriumsalz, gelöst in 10 ml Wasser/Tetrahydrofuran 1:1, erhalten.

d) Eine Lösung von 0,15 g (0,59 mMol) (3R,5R,6R)-2,2-Dimethyl-6-hydroxymethyl-penam-3-carbonsäure-natriumsalz in 10 ml Wasser/Tetrahydrofuran 1:1 wird mit o,172 g (0,88 mMol) 90%iger m-Chlorperbenzoesäure bei Raumtemperatur versetzt. Nach 1 1/2stündigem Rühren wird mit wässriger Natriumthiosulfatlösung versetzt. Die wässrige Phase wird mit 1-N Natronlauge auf pH = 6,8 eingestellt und lyophilisiert. Die Titelverbindung wird durch Chromatographie auf $UPC_{12}$-Platten gereinigt (Laufmittel, $H_2O$, Rf = 0.58).

e) 821 mg (1,58 mMol) (3R,5R,6R)-6-Benzyloxymethyl-2,2-dimethyl-penam-3-carbonsäure-diphenylmethylester-1,1-dioxid werden in 10 ml Tetrahydrofuran gelöst, mit 400 mg Pd/C 10% versetzt und unter Normaldruck bei Raumtemperatur hydriert. Nach 6 Stunden Reaktionsdauer wird vom Katalysator abfiltriert und das Filtrat am Rotationsverdampfer eingeengt. Der Rückstand wird in Wasser/Aethylacetat aufgenommen und mit wässriger gesättigter Natriumbicarbonatlösung auf pH = 6,5 eingestellt. Die wässrige Phase wird dreimal mit Aethylacetat extrahiert. Die organischen Phasen werden zweimal mit Wasser gewaschen. Nach Lyophilisation der vereinigten Wasserphasen wird die Titelverbindung als weisses Pulver erhalten. Das IR-Spektrum ist mit demjenigen von Beispiel 4a) identisch.

Beispiel 5: (3R,5R,6S)-2,2-Dimethyl-6-hydroxymethyl-penam-3-carbonsäure-diphenylmethylester-1,1-dioxid

Eine Lösung von 1,5 g (3,6 mMol) (1S,3R,5R,6S)-2,2-Dimethyl-6-hydroxymethyl-penam-3-carbonsäurediphenylmethylester-1-oxid in 45 ml Chloroform wird bei Raumtemperatur mit 0,73 g 90%iger m-Chlorpenbenzoesäure versetzt. Nach 1 1/2-stündigem Rühren wird mit wässriger Natriumthiosulfat-, wässriger Natriumbicarbonat- und wässriger Natriumchloridlösung aufgearbeitet. Die wässrigen Phasen werden zweimal mit Chloroform extrahiert, die vereinigten organischen Lösungen mit Natriumsulfat getrocknet und am Rotationsverdampfer eingeengt. Der Rückstand wird am Hochvakuum getrocknet und durch Chromatographie an 16 g Silicagel mit dem Laufmittel Toluol/Aethylacetat 3:1 gereinigt. Man erhält die reine Titelverbindung.
DC: Silicagel, Toluol/Aethylacetat 3:1, Rf = 0,22;
IR-Spektrum: Absorptionsbanden bei 2,76; 5,55; 5,67 μ ($CH_2Cl_2$).

Beispiel 6: (3R,5R,6S)-2,2-Dimethyl-6-hydroxymethyl-penam-3-carbonsäure-1,1-dioxid-natriumsalz

Eine Lösung von 0,82 g (1,9 mMol) (3R,5R,6S)-2,2-Dimethyl-6-hydroxymethyl-penam-3-carbonsäure-diphenylmethylester-1,1-dioxid in 25 ml Tetrahydrofuran wird in Gegenwart von Pd/C 10% unter Normaldruck bei Raumtemperatur hydriert. Nach 8 Stunden Reaktionsdauer wird vom Katalysator abfiltriert und das Filtrat am Rotationsverdampfer eingeengt. Der Rückstand wird in Wasser/Aethylacetat aufgenommen und langsam mit 1,9 ml 1-N NaOH versetzt (pH∿6,9). Die wässrige Phase wird dreimal mit Aethylacetat extrahiert. Nach Lyophilisation der Wasserphase wird die Titelverbindung als weisses Pulver erhalten.
DC: $UPC_{12}$-Platten, $H_2O$, Rf = 0,58;
IR-Spektrum (KBr): Absorptionsbanden bei 2,9; 5.63; 6,20 μ.

Beispiel 7: (1R,3R,5R,6R)- und (1S,3R,5R,6R)-2,2-Dimethyl-6-tert.-butyl-dimethylsilyloxymethyl-penam-3-carbonsäure-diphenylmethylester-1-oxid

24,3 g (58,9 mMol) eines Gemisches von (1R,3R,5R,6R)- und (1S,3R,5R,6R)-2,2-Dimethyl-6-hydroxymethyl-penam-3-carbonsäure-diphenylmethylester-1-oxid werden in 97 ml Dimethylformamid gelöst und bei Raumtemperatur unter Stickstoff mit 18,31 g (117,8 mMol) tert.Butyldimethylchlorsilan (97%ig) und 16.2 g (235,6 mMol) Imidazol versetzt. Nach 70 Minuten Rühren wird mit Aethylacetat verdünnt und die organische Phase dreimal mit wässriger Natriumchloridlösung gewaschen. Die Aethylacetatlösung wird mit Natriumsulfat getrocknet und am Rotationsverdampfer eingeengt. Das Rohprodukt wird an 1,1 kg Silicagel mit dem Laufmittelsystem Toluol/Aethylacetat 5:1 chromatographiert. Die beiden Titelverbindungen werden in der angegebenen Reihenfolge eluiert:

1. (1S,3R,5R,6R)-Isomer DC: Silicagel, Toluol/Aethylacetat 5:1, Rf = 0,54

   IR-Spektrum ($CH_2Cl_2$): Absorptionsbanden bei 3,42; 5,61; 5,71 μ,

2. (1R,3R,5R,6R)-Isomer DC: Silicagel, Toluol/Aethylacetat 5:1 RF = 0.4

   IR-Spektrum ($CH_2Cl_2$): Absorptionsbanden bei 3,42; 5,61; 5,71 μ.

Beispiel 8: (1S,3R,5R,6R)-2,2-Dimethyl-6-hydroxymethyl-penam-3-carbonsäure-diphenylmethylester-1-oxid

Eine Lösung von 3,13 g (5,9 mMol) (1S,3R,5R,6R)-2,2-Dimethyl-6-tert.butyl-dimethylsilyloxymethyl-penam-3-carbonsäure-diphenylmethylester-1-oxid in einem Gemisch von 46 ml Tetrahydrofuran, 93,9 ml Essigsäure und 31 ml Wasser wird während 7 Stunden auf 50°C erwärmt. Die Reaktionslösung wird am Rotationsverdampfer

eingeengt und der ölige Rückstand in Aethylacetat aufgenommen. Die organische Phase wird mit wässriger Natriumbicarbonatlösung und zweimal mit gesättigter, wässriger Natriumchloridlösung gewaschen. Nach dem Trocknen der organischen Phase über Natriumsulfat wird die Lösung am Rotationsverdampfer eingeengt und das Rohprodukt an 76 g Silicagel mit dem Laufmittelsystem Toluol/Aethylacetat 1:1 gereinigt. Man erhält die reine Titelverbindung.
DC: Silicagel, Toluol/Aethylacetat 1:1, Rf = 0.15;
IR-Spektrum ($CH_2Cl_2$): Absorptionsbanden bei 2,75; 2,95; 5,57; 5,68 µ.

Die Verbindung kann analog Beispiel 3 in das 1,1-Dioxid übergeführt werden.

Beispiel 9: (1R,3R,5R,6R)-2,2-Dimethyl-6-hydroxymethyl-penam-3-carbonsäure-diphenylmethylester-1-oxid

Analog Beispiel 8 kann das (1R,3R,5R,6R)-2,2-Dimethyl-6-tert.butyl-dimethylsilyloxymethyl-penam-3-carbonsäure-diphenylmethylester-1-oxid in die Titelverbindung übergeführt werden.
DC: Silicagel, Toluol/Aethylacetat 1:1, Rf = 0,16;
IR-Sepktrum ($CH_2Cl_2$): Absorptionsbanden bei 2,75; 2,95;, 5,59; 5,69 µ.

Beispiel 10: (1S,3R,5R,6R)-2,2-Dimethyl-6-hydroxymethyl-penam-3-carbonsäure-1-oxid-natriumsalz

Eine Lösung von 1,62 g (3,92 mMol) (1S,3R,5R,6R)-2,2-Dimethyl-6-hydroxymethyl-penam-3-carbonsäure-diphenylmethylester-1-oxid in 8 ml Tetrahydrofuran wird nach Zugabe von 1,95 g Pd/C 10% bei Raumtemperatur hydriert. Nach 5 Stunden werden weitere 2 g Pd/C 10% zugefügt und während 22 Stunden weiterhydriert. Dann wird durch Hyflo filtriert und die klare Lösung am Rotationsverdampfer

eingeengt. Der Rückstand wird mit Aethylacetat und Wasser versetzt. Unter pH-Messung wird langsam 1-N NaOH zugetropft bis pH = 6,9 erreicht ist. Die Aethylacetatphase wird abgetrennt und die wässrige Phase lyophilisiert. Das Natriumsalz der Titelverbindung wird als weisses Pulver erhalten.

DC: $UPC_{12}$-Platten, $H_2O$, Rf = 0,62;

IR-Spektrum (Nujol): Absorptionsbanden bei 3,05; 5,60; 6,20 μ.

Beispiel 11: (1R,3R,5R,6R)-2,2-Dimethyl-6-hydroxymethyl-penam-3-carbonsäure

Eine Lösung von 638 mg (1,54 mMol) (1R,3R,5R,6R)-2,2-Dimethyl-6-hydroxymethyl-penam-3-carbonsäure-diphenylmethylester in 8 ml Tetrahydrofuran wird nach Zugabe von 766 mg Pd/C 10% bei Raumtemperatur hydriert. Nach 23 Stunden erfolgt Zugabe von 766 mg Pd/C 10%, worauf weitere 7,5 Stunden hydriert wird. Die Reaktionslösung wird durch Hyflo filtriert und am Rotationsverdampfer eingeengt. Der Rückstand kristallisiert aus Methylenchlorid.

DC: $UPC_{12}$-Platten, $H_2O$, Rf = 0,64;

IR-Spektrum (Nujol): Absorptionsbanden bei 2,8; 5,60; 5,75 μ.

Beispiel 12: (3R,5R,6R)-2,2-Dimethyl-6-tert.butyl-dimethylsilyloxymethyl-penam-3-carbonsäure-diphenylmethylester

a) Eine Lösung von 4,0 g (7,59 mMol) (1S,3R,5R,6R)-2,2-Dimethyl-6-tert.butyl-dimethylsilyloxymethyl-penam-3-carbonsäure-diphenylmethylester-1-oxid in 80 ml Methylenchlorid und 20 ml Dimethylacetamid wird bei 0°C unter $N_2$ mit 2,8 ml (30,36 mMol) Phosphortribromid versetzt. Nach 30 Minuten Reaktionsdauer wird mit gekühlter Natriumbicarbonatlösung versetzt. Die organische Phase wird abgetrennt, zweimal mit gesättigter, wässriger Natriumchloridlösung gewaschen und über Natriumsulfat getrocknet. Die Lösung wird am Rotationsverdampfer eingeengt und der Rückstand an 45 g Silicagel mit dem Lösungsmittelsystem Toluol/Aethylacetat 10:1 chromatographiert.
DC: Silicagel, Toluol/Aethylacetat 10:1, Rf = 0,61;
IR-Spektrum ($CH_2Cl_2$): Absorptionsbanden bei 3,4; 5,64; 5,72 µ.

b) Die gleiche Titelverbindung wird analog a) ausgehend von (1R,3R,5R,6R)-2,2-Dimethyl-6-tert.butyl-dimethylsilyloxymethyl-penam-3-carbonsäure-diphenylmethylester-1-oxid erhalten.

Beispiel 13: (3R,5R,6R)-2,2-Dimethyl-6-hydroxymethyl-penam-3-carbonsäure-diphenylmethylester

a) Eine Lösung von 3,4 g (6,65 mMol) (3R,5R,6R)-2,2-Dimethyl-6-tert.butyl-dimethylsilyloxymethyl-penam-3-carbonsäure-diphenylmethylester in einem Gemisch von 52 ml Tetrahydrofuran, 105 ml Essigsäure und 35 ml Wasser wird während 7 Stunden auf 50°C erwärmt. Die Reaktionslösung wird am Rotationsverdampfer eingeengt und der ölige Rückstand in Aethylacetat aufgenommen. Die organische Phase wird mit wässriger Natriumbicarbonatlösung und zweimal mit gesättigter Natriumchloridlösung gewaschen. Nach dem Trocknen der organischen Phase über Natriumsulfat wird die Lösung am Rotationsverdampfer eingeengt. Das Rohprodukt wird an 50 g Silicagel mit dem Laufmittelsystem Toluol/Aethylacetat 2:1 gereinigt. Man erhält die Titelverbindung vom Schmelzpunkt 120,5°C.
DC: Silicagel, Toluol/Aethylacetat 2:1, Rf = 0,37;
IR-Spektrum ($CH_2Cl_2$): Absorptionsbanden bei 2,8; 5,65; 5,7 µ.

b) Die gleiche Verbindung kann auch wie folgt erhalten werden:
Eine Lösung von 4,4 g (9,2 mMol) (3R,5R,6R)-6-Brom-2,2-dimethyl-6-hydroxymethyl-penam-3-carbonsäure-diphenylmethylester in 40 ml Toluol und 3,2 ml (12 mMol) Tributylzinnhydrid wird unter Stickstoffatmosphäre während 2,5 Stunden auf 80° erwärmt. Dann wird das Reaktionsgemisch eingeengt und der Rückstand aus Hexan kristallisiert. Nach Umkristallisation aus Methylenchlorid/Hexan erhält man die Titelverbindung.

c) Die Titelverbindung wird in analoger Weise, wie unter b) beschrieben, auch ausgehend von (3R,5R,6S)-6-Brom-2,2-dimethyl-6-hydroxymethyl-penam-3-carbonsäure-diphenylmethylester erhalten.

Beispiel 14: (3R,5R,6R)-2,2-Dimethyl-6-hydroxymethyl-penam-3-carbonsäure-natriumsalz

Eine Lösung von 1,9 g (4,98 mMol) (3R,5R,6R)-2,2-Dimethyl-6-hydroxymethyl-penam-3-carbonsäure-diphenylmethylester in 82 ml Tetrahydrofuran wird nach Zugabe von 2,1 g Pd/C 10% bei Raumtemperatur hydriert. Nach 16 Stunden und 24 Stunden werden noch je 2,1 g Pd/C 10% zugegeben. Nach insgesamt 40 Stunden Reaktionsdauer wird die Hydriermischung durch Hyflo filtriert und am Rotationsverdampfer eingeengt. Der ölige Rückstand wird in Wasser/Aethylacetat aufgenommen und vorsichtig mit 2,7 ml 1-N NaOH versetzt (pH 6,9). Die wässrige Phase wird dreimal mit Aethylacetat extrahiert und anschliessend lyophilisiert. Man erhält die Titelverbindung als weisses Pulver.
DC: $UPC_{12}$-Platten, $H_2O$, Rf = 0,45;
IR-Spektrum (Nujol): Absorptionsbanden bei 2,93; 5,63; 6,27 μ.

Beispiel 15: (3R,5R,6R und 6S)-6-Brom-2,2-dimethyl-6-hydroxymethyl-penam-3-carbonsäure-diphenylmethylester

Eine Lösung von 50 g (95 mMol) (3R,5R)-6,6-Dibrom-2,2-dimethyl-penam-3-carbonsäure-diphenylmethylester in 370 ml Tetrahydrofuran wird auf -78° gekühlt und mit 52,2 ml (95 mMol) einer 1,82-molaren Lösung von Methylmagnesiumbromid in Diäthyläther versetzt. Nach 10 Minuten bei -78° wird unter gutem Rühren Formaldehydgas über die Lösung geleitet.

Nach 2 Stunden zeigt eine DC-Probe vollständigen Umsatz an. Man fügt 25 ml Eisessig hinzu und engt die Lösung am Rotationsverdampfer ein. Anschliessend wird der Rückstand in Aethylacetat aufgenommen und mit wässriger Natriumbicarbonat-Lösung und Kochsalz-Lösung gewaschen. Die organischen Phasen werden über Natriumsulfat getrocknet und am Rotationsverdampfer eingeengt. Der Rückstand wird an 480 g Silicagel mit dem Lösungsmittelsystem Toluol/Aethylacetat (6:1) chromatographiert. Man erhält ein Gemisch der Titelverbindungen im Verhältnis 3(6R):1(6S).

(6R)-Isomer: DC:Silicagel, Toluol/Aethylacetat (6:1) Rf = 0,36
IR-Spektrum ($CH_2Cl_2$): Absorptionsbänden bei 2,75; 5,57; 5,70 μ. Smp.: 144°-146°.

(6S)-Isomer: DC:Silicagel, Toluol/Aethylacetat (6:1), Rf = 0,30
IR-Spektrum ($CH_2Cl_2$): Absorptionsbanden bei 2,75; 5,57; 5,70 μ. Smp.: 158°-161°.

Beispiel 16: (3R,5R,6R)-2,2-Dimethyl-6-hydroxymethyl-penam-3-carbonsäure-1,1-dioxid

a) 2 g (4,66 mMol) (3R,5R,6R)-2,2-Dimethyl-6-hydroxymethyl-penam-3-carbonsäure-diphenylmethylester-1,1-dioxid in 20 ml Tetrahydrofuran wird mit 1 g Pd/C 10% versetzt und unter Normaldruck, bei Raumtemperatur hydriert. Nach einer Stunde Reaktionsdauer wird vom Katalysator abfiltriert und das Filtrat am Rotationsverdampfer eingeengt. Der Rückstand wird aus Methylenchlorid/Cyclohexan kristallisiert. Man erhält die Titelverbindung vom Smp. 150° (Zersetzung)

b) Die gleiche Verbindung kann auch wie folgt erhalten werden:
2 g (3,86 mMol) (3R,5R,6R)-6-Benzyloxymethyl-2,2-dimethyl-penam-3-carbonsäure-diphenylmethylester-1,1-dioxid werden in 20 ml Tetrahydrofuran gelöst, mit 1 g Pd/C 10% versetzt und eine Stunde unter Normaldruck bei Raumtemperatur hydriert. Nach 16 Stunden Reaktionsdauer wird vom Katalysator abfiltriert und das Filtrat am Rotationsverdampfer eingeengt. Der Rückstand wird aus Methylenchlorid/Cyclohexan kristallisiert.

Beispiel 17: (3R,5R,6R)-6-Benzyloxymethyl-6-brom-2,2-dimethyl-penam-3-carbonsäure-diphenylmethylester

a) Eine Lösung von 10 g (19 mMol) (3R,5R)-6,6-Dibrom-2,2-dimethyl-penam-3-carbonsäure-diphenylmethylester in 50 ml Tetrahydrofuran wird auf -30° gekühlt und langsam mit 10,4 ml (19 mMol) einer 1,8-molaren Lösung von Methylmagnesiumbromid in Diäthyläther versetzt. Nach 5 Minuten bei -30° werden 5,2 ml (38 mMol) Benzylchlormethyläther zugegeben. Das Reaktionsgemisch wird während einer Stunde bei -30° gerührt und anschliessend innerhalb einer 3/4 Stunde auf 0° erwärmt. Dann wird die Lösung am Rotationsverdampfer eingeengt, der Rückstand in Aethylacetat aufgenommen und mit wässeriger Natriumbicarbonatlösung und Kochsalzlösung gewaschen. Die organischen Phasen werden über Natriumsulfat getrocknet und an Rotationsverdampfer eingeengt. Der Rückstand wird an 130 g Silicagel mit Toluol als Laufmittel chromatographiert. Man erhält nach dem Abdampfen des Lösungsmittels die Titelverbindung.

DC: Silicagel, Toluol, Rf = 0.16

IR-Spektrum ($CH_2Cl_2$): Absorptionsbanden bei 3,45; 5,60; 5,74; 6,28 μ.

b) Die gleiche Verbindung kann auch wie folgt hergestellt werden: Eine Lösung von 5 g (9,5 mMol) (3R,5R)-6,6-Dibrom-2,2-dimethyl-penam-3-carbonsäure-diphenylmethylester in 25 ml Tetrahydrofuran wird auf -78° gekühlt und langsam mit 5 ml (9,5 mMol) einer 1,9-molaren Lösung von Methylmagnesiumbromid in Diäthyläther versetzt. Nach 5 Minuten bei -78° werden 3,8 g Benzylbrommethyläther zugegeben. Das Reaktionsgemisch wird innerhalb von 40 Minuten auf Raumtemperatur erwärmt und anschliessend am Rotationsverdampfer eingeengt. Der Rückstand wird in

Aethylacetat aufgenommen und mit wässeriger Natriumbicarbonatlösung und Kochsalzlösung gewaschen. Die organischen Phasen werden über Natriumsulfat getrocknet und am Rotationsverdampfer eingeengt. Der Rückstand wird an 70 g Silicagel mit Toluol als Laufmittel chromatographiert. Die physikalisch-chemischen Daten der erhaltenen Titelverbindung sind mit den unter a) genannten identisch.

Beispiel 18: (3R,5R,6R)-6-Benzyloxymethyl-2,2-dimethyl-penam-3-carbonsäure-diphenylmethylester

Eine Lösung von 2,4 g (4,24 mMol) (3R,5R,6R)-6-Benzyloxymethyl-6-brom-2,2-dimethyl-penam-3-carbonsäure-diphenylmethylester und 1,5 ml (5,6 mMol) Tributylzinnhydrid in 20 ml Toluol wird unter Stickstoffatmosphäre während 80 Minuten auf 82° erwärmt. Dann wird das Reaktionsgemisch eingeengt und der Rückstand aus Isooctan kristallisiert. Nach Umkristallisation aus Methylenchlorid/Isooctan erhält man die Titelverbindung vom Smp. 78°-80°.

DC: Silicagel, Toluol/Aethylacetat (20:1), Rf = 0,27

IR-Spektrum ($CH_2Cl_2$): Absorptionsbanden bei 3,44; 5,65; 5,73 μ.

Beispiel 19: (3R,5R,6R)-6-Benzyloxymethyl-2,2-dimethyl-penam-3-carbonsäure-diphenylmethylester-1,1-dioxid

1,68 g (3,45 mMol) (3R,5R,6R)-6-Benzyloxymethyl-2,2-dimethyl-penam-3-carbonsäure-diphenylmethylester werden in 16 ml Chloroform gelöst und mit 1,43 g (8,3 mMol) 90%iger m-Chlorperbenzoesäure bei Raumtemperatur versetzt. Nach 50 Minuten wird nacheinander mit wässriger Natriumthiosulfat-, wässriger Natriumbicarbonat- und wässriger Natriumchloridlösung aufgearbeitet. Die wässrigen Phasen werden zweimal mit Chloroform nachextrahiert. Die vereinigten organischen Lösungen werden mit Natriumsulfat getrocknet und am Rotationsverdampfer eingeengt. Der Rückstand wird im Hochvakuum getrocknet und durch präparative Dünnschichtchromatographie mit dem Lösungsmittelsystem Toluol/Aethylacetat (20:1) gereinigt. Man erhält die kristalline Titelverbindung vom Smp. 124°-126,5°.

DC: Silicagel, Toluol/Aethylacetat (20:1), Rf = 0.52
IR-Spektrum ($CH_2Cl$): Absorptionsbanden bei 3,45; 5,57 ; 5,71 µ.

Beispiel 20: (3R,5R,6R)-6-Brom-2,2-dimethyl-6-methoxymethyl-penam-3-carbonsäure-diphenylmethylester

Eine Lösung von 10 g (19 mMol) (3R,5R)-6,6-Dibrom-2,2-dimethyl-penam-3-carbonsäure-diphenylmethylester in 50 ml Tetrahydrofuran wird auf -78° gekühlt und mit 9,2 ml (19 mMol) Methylmagnesiumbromid in Diäthyläther versetzt. Nach 5 Minuten bei -78° werden 2,82 ml Chlordimethyläther zugegeben. Das Reaktionsgemisch wird auf -20° erwärmt und nach 95 Minuten am Rotationsverdampfer eingeengt. Der Rückstand wird in Aethylacetat aufgenommen und mit wässeriger Natriumbicarbonatlösung und Kochsalzlösung gewaschen. Die organischen Phasen werden über Natriumsulfat getrocknet und am Rotationsverdampfer eingeengt. Der Rückstand wird an 30 g Silicagel mit Toluol als Laufmittel chromatographiert. Man erhält die reine Titelverbindung.
DC: Silicagel, Toluol/Aethylacetat (20:1), Rf = 0.34.
IR-Spektrum ($CH_2Cl_2$): Absorptionsbanden bei 5,62; 5,73 µ.

Beispiel 21: (3R,5R,6R)-2,2-Dimethyl-6-methoxymethyl-penam-3-carbonsäure-diphenylmethylester

Eine Lösung von 7,45 g (15,1 mMol) (3R,5R,6R)-6-Brom-2,2-dimethyl-6-methoxymethyl-penam-3-carbonsäure-diphenylmethylester und 5,22 ml (19,7 mMol) Tributylzinnhydrid in 72 ml Toluol wird unter Stickstoffatmosphäre während 45 Minuten auf 82° erwärmt. Dann wird das Reaktionsgemisch eingeengt und aus Hexan kristallisiert. Nach Umkristallisation aus Methylenchlorid/Hexan erhält man die Titelverbindung vom Smp. 84°.
DC: Silicagel, Toluol/Aethylacetat (20:1), Rf = 0,31.
IR-Spektrum ($CH_2Cl_2$): Absorptionsbanden bei 5,63; 5,72 µ.

Beispiel 22: (3R,5R,6R)-2,2-Dimethyl-6-methoxymethyl-penam-3-carbonsäure-diphenylmethylester-1,1-dioxid

4,8 g (11,6 mMol) (3R,5R,6R)-2,2-Dimethyl-6-methoxymethyl-penam-3-carbonsäure-diphenylmethylester werden in 48 ml Chloroform gelöst und bei 35° mit 5,5 g (28,9 mMol) 90%iger m-Chlorperbenzoesäure versetzt. Nach 2,5 Stunden wird mit wässeriger Natriumbisulfitlösung und Natriumbicarbonatlösung aufgearbeitet. Die wässerigen Phasen werden zweimal mit Chloroform nachextrahiert. Die vereinigten organischen Lösungen werden mit Natriumsulfat getrocknet und am Rotationsverdampfer eingeengt. Der Rückstand wird aus Aethylacetat/Hexan kristallisiert und ergibt die Titelverbindung vom Smp. 120-126°.
DC: Silicagel, Toluol/Aethylacetat, Rf = 0.29.
IR-Spektrum ($CH_2Cl_2$): Absorptionsbanden bei 5,55; 5,69 µ.

Beispiel 23: (3R,5R,6R)-2,2-Dimethyl-6-methoxymethyl-penam-3-carbonsäure-1,1-dioxid

4,7 g (10,6 mMol) (3R,5R,6R)-2,2-Dimethyl-6-methoxymethyl-penam-3-carbonsäure-diphenylmethylester-1,1-dioxid werden in 52 ml Tetrahydrofuran gelöst, mit 2 g Pd/C 10% versetzt und unter Normaldruck bei Raumtemperatur hydriert. Nach einer Stunde Reaktionsdauer wird vom Katalysator abfiltriert und das Filtrat am Rotationsverdampfer eingeengt. Der Rückstand wird aus Aethylacetat/Hexan kristallisiert und ergibt die Titelverbindung vom Smp. 145°.
DC: $UPC_{12}$-Platten, Wasser/Acetonitril (95:5), Rf = 0.37.
IR-Spektrum ($CH_2Cl_2$): Absorptionsbanden bei 5,55; 5,75, 7,5 µ.

Beispiel 24: (3R,5R,6R)-2,2-Dimethyl-6-methoxymethyl-penam-3-carbonsäure-1'-äthoxycarbonyloxyäthylester-1,1-dioxid

12 g Natriumjodid werden in 37 ml Aceton gelöst und mit 2,75 ml Aethyl-1-chloräthylcarbonat versetzt. Das Gemisch wird bei Raumtemperatur während 3 Stunden gerührt. Anschliessend wird die Lösung auf 150 ml Methylenchlorid getropft und von den ausgefallenen anorganischen Salzen abfiltriert. Die Methylenchloridlösung wird bis auf 10 ml eingeengt und bei 0° zu einer Lösung von 2,5 g (8,05 mMol)(3R,5R,6R)-2,2-Dimethyl-6-hydroxymethyl-penam-3-carbonsäure-1,1-dioxid-natriumsalz in 40 ml Dimethylacetamid gegeben. Dann wird während 3 Stunden bei 0° gerührt, anschliessend mit Aethylacetat verdünnt und dreimal mit Wasser gewaschen. Die organischen Phasen werden über Natriumsulfat getrocknet und am Rotationsverdampfer eingeengt. Das Rohprodukt wird an 30 g Silicagel mit dem Laufmittelsystem Toluol/Aethylacetat (2:1) gereinigt. Man erhält die Titelverbindung als weissen Schaum.
DC: Silicagel, Toluol/Aethylacetat (2:1), Rf = 0,48
IR-Spektrum ($CH_2Cl_2$): Absorptionsbanden bei 2,75; 5,55; 5,65 μ.

Beispiel 25: (3R,5R,6R)-2,2-Dimethyl-6-hydroxymethyl-penam-3-carbonsäure-pivaloyloxymethylester-1,1-dioxid

6 g Natriumjodid werden in 20 ml Aceton gelöst und mit 1,5 ml Pivalinsäurechlormethylester versetzt. Das Gemisch wird bei Raumtemperatur während 3 Stunden gerührt und anschliessend auf 75 ml Methylenchlorid getropft. Die ausgefallenen anorganischen Salze werden abfiltriert. Die Methylenchloridlösung wird bis auf 10 ml eingeengt und zu einer Lösung von 1,3 g (4 mMol)(3R,5R,6R)-2,2-Dimethyl-6-hydroxymethyl-penam-3-carbonsäure-1,1-dioxid-natriumsalz in 20 ml N,N-Dimethylacetamid bei 0° gegeben. Dann wird während 3 Stunden bei 0° gerührt, anschliessend mit Aethylacetat verdünnt und dreimal mit Wasser gewaschen. Die organische Phase wird über Natriumsulfat getrocknet und am Rotationsverdampfer eingeengt. Das Rohprodukt wird an 30 g Silicagel mit dem Laufmittelsystem Toluol/Aethylacetat (3:1) gereinigt. Man erhält die Titelverbindung als weissen Schaum.
DC: Silicagel, Toluol/Aethylacetat (3:1), Rf = 0.2; IR-Spektrum ($CH_2Cl_2$): Absorptionsbanden bei 2.75; 5.55; 5.62; 5.68 μ.

Beispiel 26: (3R,5R,6R)-2,2-Dimethyl-6-benzyloxymethyl-penam-3-carbonsäure-diphenylmethylester-1,1-dioxid

Eine Lösung von 237 g (0,487 Mol)(3R,5R,6R)-2,2-Dimethyl-6-benzyloxymethyl-penam-3-carbonsäure-diphenylmethylester in 237 ml Tetrahydrofuran und 1 l Eisessig werden mit 169,4 g Kaliumpermanganat versetzt,

wobei die Reaktionstemperatur auf 36° steigt. Bei dieser Temperatur wird während 2 3/4 Std. gerührt, worauf nacheinander 100 ml Wasserstoffperoxidlösung (30%) und 4 l Wasser zugegeben werden. Die ausgefallene Titelverbindung wird mit Wasser gewaschen, in Aethylacetat aufgelöst und nacheinander mit Wasser, wässriger Natriumbicarbonatlösung und Wasser gewaschen. Die organische Phase wird über Natriumsulfat getrocknet und mit Aktivkohle gereinigt. Die erhaltene Titelverbindung wird durch Kristallisation aus Aethylacetat/Hexan gereinigt.

Beispiel 27: (3R,5R,6S und 6R)-2,2-Dimethyl-6-brom-6-hydroxymethyl--penam-3-carbonsäure-diphenylmethylester-1,1-dioxid

Eine Lösung von 30 g (53,7 mMol) (3R,5R)-2,2-Dimethyl-6,6-dibrom-penam-3-carbonsäure-diphenylmethylester-1,1-dioxid in 150 ml Tetrahydrofuran wird auf -78° gekühlt und mit 28,2 ml (53,7 mMol) einer 1,9 M Lösung von Methylmagnesiumbromid in Diäthyläther versetzt. Nach 10 Minuten bei -78° wird unter Rühren Formaldehydgas über die Lösung geleitet. Nach 2 Std. werden 25 ml Eisessig zugefügt und die Lösung am Rotationsverdampfer eingeengt. Der Rückstand wird in Aethylacetat aufgenommen und mit wässriger Natriumbicarbonatlösung und Kochsalzlösung gewaschen. Die organischen Phasen werden über Natriumsulfat getrocknet und am Rotationsverdampfer eingeengt. Der Rückstand wird an 250 g Silicagel mit dem Lösungsmittelsystem Toluol/Aethylacetat 6:1 chromatographiert und man erhält ein Gemisch der Titelverbindungen.
Gemisch: DC: Silicagel, Tol./Aethylacetat 6:1 Rf = 0,28
IR-Spektrum ($CH_2Cl_2$): Absorbtionsbanden bei 2,74; 5,53; 5,7 μ.

Beispiel 28: (3R,5R,6R)-2,2-Dimethyl-6-hydroxymethyl-penam-3-carbonsäure-diphenylmethylester-1,1-dioxid

Eine Lösung von 25 g (49,2 mMol) eines Gemisches von (3R,5R,6S und 6R)-2,2-Dimethyl-6-brom-6-hydroxymethyl-penam-3-carbonsäure-diphenylmethylester-1,1-dioxid und 15,7 ml (59 mMol) Tributylzinnhydrid in 250 ml Toluol wird unter Stickstoffatmosphäre während 2,5 Stunden auf 80° erwärmt. Das Reaktionsgemisch wird eingeengt und der Rückstand

aus Tetrahydrofuran/Hexan auskristallisiert. Smp. 70°-75°.

Beispiel 29: (3R,5R,6R)-2,2-Dimethyl-6-brom-6-benzyloxymethyl-penam-3-carbonsäure-diphenylmethylester-1,1-dioxid

Eine Lösung von 5 g (8,96 mMol) (3R,5R,6S und 6R)-2,2-Dimethyl-6,6-dibrom-penam-3-carbonsäure-diphenylmethylester-1,1-dioxid in 25 ml Tetrahydrofuran wird auf -78° gekühlt und langsam mit 4,7 ml (8,96 mMol) einer 1,9-molaren Lösung von Methylmagnesiumbromid in Diäthyläther versetzt. Nach 5 Minuten bei -78° werden 3,6 g Benzyloxymethylbromid zugegeben. Das Reaktionsgemisch wird innerhalb 40 Minuten auf Raumtemperatur erwärmt und anschliessend am Rotationsverdampfer eingeengt. Der Rückstand wird in Aethylacetat aufgenommen und mit wässriger Natriumbicarbonatlösung und Kochsalzlösung gewaschen. Die organischen Phasen werden über Natriumsulfat getrocknet und am Rotationsverdampfer eingeengt. Der Rückstand wird an 70 g Silicagel mit Toluol chromatographiert und man erhält die Titelverbindung.
DC: Silicagel, Toluol, Rf = 0,13
IR-Spektrum ($CH_2Cl_2$): Absorptionsbanden bei 5,53; 5,74 μ.

Beispiel 30: (3R,5R,6R)-2,2-Dimethyl-6-benzyloxymethyl-penam-3-carbonsäure-diphenylmethylester-1,1-dioxid

Eine Lösung von 2,4 g (4 mMol) (3R,5R,6R)-2,2-Dimethyl-6-brom-6-benzyloxymethyl-penam-3-carbonsäure-diphenylmethylester-1,1-dioxid und 1,5 ml (5,6 mMol) Tributylzinnhydrid in 20 ml Toluol wird unter Stickstoffatmosphäre während 80 Minuten auf 82° erwärmt. Dann wird das Reaktionsgemisch eingeengt und der Rückstand aus Isooctan kristallisiert. Nach Umkristallisation aus Aethylacetat/Hexan erhält man die Titelverbindung vom Smp. 119-123°.
DC: Silicagel, Toluol/Aethylacetat 6:1, Rf = 0,52
IR-Spektrum ($CH_2Cl_2$): Absorptionsbanden bei 3,44; 5,57; 5,73 μ.

Pharmazeutische Präparate

Beispiel A Trockenampullen oder Vials enthaltend 1 g (3R,5R,6R)-2,2-Dimethyl-6-hydroxymethylpenam-3-carbonsäure-1,1-dioxid-natriumsalz als Wirksubstanz werden wie folgt hergestellt:

Zusammensetzung (für 1000 Ampullen oder Vials):

| | |
|---|---|
| Wirksubstanz | 1 000 g |
| Mannit | 100 g |
| | 1 100 g |

Die Komponenten werden homogen gemischt und je 1,1 g der Mischung unter aseptischen Bedingungen in eine Ampulle oder ein Vial abgefüllt. Die Ampullen oder Vials werden verschlossen und geprüft.

Auf die gleiche Weise werden Ampullen oder Vials mit entsprechenden Mengen anderer erfindungsgemässer Wirkstoffkomponenten hergestellt.

Beispiel B Trockenampullen oder Vials enthaltend 2 g (3R,5R,6R)-2,2-Dimethyl-6-hydroxymethylpenam-3-carbonsäure-1,1-dioxid-natriumsalz als Wirksubstanz A und 0,2 g Cefotiam als Wirksubstanz B werden wie folgt hergestellt:

Zusammensetzung (für 1000 Ampullen oder Vials):

| | |
|---|---|
| Wirksubstanz A | 1 000 g |
| Wirksubstanz B | 100 g |
| Mannit | 100 g |
| | 1 200 g |

Die Komponenten werden homogen gemischt und je 2,4 g der Mischung unter aseptischen Bedingungen in eine Ampulle oder ein Vial abgefüllt. Die Ampullen oder Vials werden verschlossen und geprüft.

Auf die gleiche Weise werden Ampullen oder Vials mit entsprechenden Mengen anderer erfindungsgemässer Wirkstoffkomponenten hergestellt.

Beispiel C Trockenampullen oder Vials enthaltend 2,0 g (3R,5R,6R)-2,2-Dimethyl-6-hydroxymethylpenam-3-carbonsäure-1,1-dioxid-natriumsalz als Wirksubstanz A und 0,4 g Cefotiam als Wirksubstanz B werden wie folgt hergestellt:

Zusammensetzung (für 1000 Ampullen oder Vials):

| | |
|---|---|
| Wirksubstanz A | 2 000 g |
| Wirksubstanz B | 400 g |
| Mannit | 200 g |
| | 2 600 g |

Die Komponenten werden homogen gemischt und je 2,6 g der Mischung unter aseptischen Bedingungen in eine Ampulle oder Vial abgefüllt. Die Ampullen oder Vials werden verschlossen und geprüft.

Auf die gleiche Weise werden Ampullen oder Vials mit entsprechenden Mengen anderer erfindungsgemässer Wirkstoffkomponenten hergestellt.

Beispiel D Trockenampullen oder Vials enthaltend 2g (3R,5R,6R)-2,2-Dimethyl-6-hydroxymethylpenam-3-carbonsäure-1,1-dioxid-natriumsalz als Wirksubstanz A und 1 g Cefotiam als Wirksubstanz B (2:1) werden wie folgt hergestellt:

Zusammensetzung (für 1000 Ampullen oder Vials):

| | |
|---|---|
| Wirksubstanz A | 2 000 g |
| Wirksubstanz B | 1 000 g |
| Mannit | 300 g |
| | 3 300 g |

Die Komponenten werden homogen gemischt und je 3,3 g der Mischung unter aseptischen Bedingungen in eine Ampulle oder Vials abgefüllt. Die Ampullen oder Vials werden geschlossen und geprüft.

Auf die gleiche Weise werden Ampullen oder Vials mit entsprechenden Mengen anderer erfindungsgemässer Wirkstoffkomponenten hergestellt.

Beispiel E Trockenampullen oder Vials enthaltend 1 g (3R,5R,6R)-2,2-Dimethyl-6-hydroxymethylpenam-3-carbonsäure-1,1-dioxid-natriumsalz als Wirksubstanz A und 1 g Cefotiam als Wirksubstanz B werden wie folgt hergestellt:

Zusammensetzung (für 1000 Ampullen oder Vials):

| | |
|---|---|
| Wirksubstanz A | 1 000 g |
| Wirksubstanz B | 1 000 g |
| Mannit | 200 g |
| | 2 200 g |

Die Komponenten werden homogen gemischt und je 2,2 g der Mischung unter aseptischen Bedingungen in eine Ampulle oder Vial abgefüllt. Die Ampullen oder Vials werden verschlossen und geprüft.

Auf die gleiche Weise werden Ampullen oder Vials mit entsprechenden Mengen anderer erfindungsgemässer Wirkstoffkomponenten hergestellt.

Beispiel F Trockenampullen oder Vials enthaltend 1 g (3R,5R,6R)-2,2-Dimethyl-6-hydroxymethylpenam-3-carbonsäure-1,1-dioxid-natriumsalz als Wirksubstanz A und 3 g Cefotiam als Wirksubstanz B werden wie folgt hergestellt:

Zusammensetzung (für 1000 Ampullen oder Vials):

| | |
|---|---|
| Wirksubstanz A | 1 000 g |
| Wirksubstanz B | 3 000 g |
| Mannit | 300 g |
| | 4 300 g |

Die Komponenten werden homogen gemischt und je 4,3 g der Mischung unter aseptischen Bedingungen in eine Ampulle oder Vials abgefüllt. Die Ampullen oder Vials werden verschlossen und geprüft.

Auf die gleiche Weise werden Ampullen oder Vials mit entsprechenden Mengen anderer erfindungsgemässer Wirkstoffkomponenten hergestellt.

Beispiel G Trockenampullen oder Vials enthaltend 0,25 g (3R,5R,6R)-2,2-Dimethyl-6-hydroxymethylpenam-3-carbonsäure-1,1-dioxid-natriumsalz als Wirksubstanz A und 1,25 g Cefotiam als Wirksubstanz B werden wie folgt hergestellt:

Zusammensetzung (für 1000 Ampullen oder Vials):

| | |
|---|---|
| Wirksubstanz A | 250 g |
| Wirksubstanz B | 1 250 g |
| Mannit | 200 g |
| | 1 700 g |

Die Komponenten werden homogen gemischt und je 1,7 g der Mischung unter aseptischen Bedingungen in eine Ampulle oder Vial abgefüllt. Die Ampullen oder Vials werden verschlossen und geprüft.

Auf die gleiche Weise werden Ampullen oder Vials mit entsprechenden Mengen anderer erfindungsgemässer Wirkstoffkomponenten hergestellt.

Beispiel H Trockenampullen oder Vials enthalten 0,25 g (3R,5R,6R)-2,2-Dimethyl-6-hydroxymethylpenam-3-carbonsäure-1,1-dioxid-natriumsalz als Wirksubstanz A und 2,5 g Cefotiam als Wirksubstanz B (1:10) werden wie folgt hergestellt:

Zusammensetzung (für 1000 Ampullen oder Vials):

| | |
|---|---|
| Wirksubstanz A | 250 g |
| Wirksubstanz B | 2 500 g |
| Mannit | 200 g |
| | 2 950 g |

Die Komponenten werden homogen gemischt und je 2,950 g der Mischung unter aseptischen Bedingungen in eine Ampulle oder Vial abgefüllt. Die Ampullen oder Vials werden verschlossen und geprüft.

Auf die gleiche Weise werden Ampullen oder Vials mit entsprechenden Mengen anderer erfindungsgemässer Wirkstoffkomponenten hergestellt.

Patentansprüche (für alle benannten Länder ausser Oesterreich)

1. 2,2-Dimethyl-penam-3-carbonsäure-1,1-dioxid-verbindungen der Formel

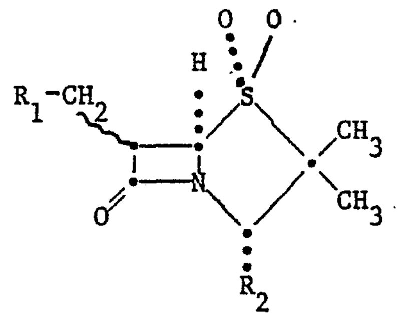

(I) ,

worin $R_1$ Hydroxy oder veräthertes oder verestertes Hydroxy darstellt und $R_2$ Carboxyl oder geschütztes Carboxyl bedeutet, und Salze von solchen Verbindungen, die eine salzbildende Gruppe aufweisen.

2. Verbindungen der Formel (I) gemäss Patentanspruch 1, worin $R_1$ Hydroxy, Niederalkoxy, z.B. Methoxy, Phenylniederalkoxy, z.B. Benzyloxy, Niederalkanoyloxy, z.B. Formyloxy oder Acetoxy, Aminothiazolylcarbonyloxy, z.B. 2-Amino-1,3-thiazol-4-ylcarbonyloxy, Aminothiadiazolylcarbonyloxy, z.B. 5-Amino-1,2,4-thiadiazol-3-yl-carbonyloxy, Niederalkoxycarbonyloxy, Carbamoyloxy, N-Mono- oder N,N-Diniederalkylcarbamoyloxy, z.B. N-Methylcarbamoyloxy oder N,N-Dimethylcarbamoyloxy, Niederalkansulfonyloxy, z.B. Methansulfonyloxy, Arensulfonyloxy, z.B. Benzolsulfonyloxy oder Toluolsulfonyloxy, oder Hydroxysulfonyloxy (oder ein Salz davon, z.B. das Natriumsalz) darstellt und $R_2$ Carboxy oder in physiologisch spaltbarer Form verestertes Carboxy, z.B. Niederalkanoyloxymethoxycarbonyl, z.B. Acetyloxymethyloxycarbonyl oder Pivaloyloxymethoxycarbonyl, Aminoniederalkanoylmethoxycarbonyl, insbesondere α-Amino-niederalkanoyloxymethoxycarbonyl, z.B. Glycyloxymethoxycarbonyl, L-Valyloxymethoxycarbonyl, L-Leucyloxymethoxycarbonyl, Phthalidyloxycarbonyl, z.B. 2-Phthalidyloxycarbonyl, 4-Crotonolactonyl oder gamma-Butyrolacton-4-yl, Indanyloxycarbonyl,

z.B. 5-Indanyloxycarbonyl, oder 1-Aethyloxycarbonyloxyäthyloxycarbonyl bedeutet, sowie die pharmazeutisch annehmbaren Salze von solchen Verbindungen, die eine salzbildende Gruppe enthalten.

3. Verbindungen der Formel (I) gemäss Patentanspruch 1, worin $R_1$ Hydroxy, Methoxy oder Benzyloxy darstellt und $R_2$ Carboxy bedeutet, sowie die pharmazeutisch annehmbaren Salze davon.

4. Verbindungen der Formel (I) gemäss Patentanspruch 1, worin $R_1$ Hydroxy ist.

5. Verbindungen der Formel (I) gemäss Patentanspruch 1, deren 6-Stellung die R-Konfiguration besitzt.

6. (3R,5R,6R)-2,2-Dimethyl-6-hydroxymethyl-penam-3-carbonsäure-diphenylmethylester-1,1-dioxid gemäss Patentanspruch 1.

7. (3R,5R,6R)-2,2-Dimethyl-6-hydroxymethyl-penam-3-carbonsäure-1,1-dioxid-natriumsalz gemäss Patentanspruch 1.

8. (3R,5R,6R)-2,2-Dimethyl-6-hydroxymethyl-penam-3-carbonsäure-pivaloyloxymethylester-1,1-dioxid gemäss Patentanspruch 1.

9. Die pharmazeutisch annehmbaren Salze der Verbindungen gemäss einem der Patentansprüche 1-5, die eine salzbildende Gruppe besitzen.

10. Pharmazeutische Präparate enthaltend eine Verbindung der Formel (I) gemäss einem der Patentansprüche 1 - 7 oder 9 oder ein pharmazeutisch annehmbares Salz einer solchen Verbindung, die eine salzbildende Gruppe besitzt.

11. Pharmazeutische Präparate enthaltend die Verbindung gemäss Patentanspruch 8.

12. Pharmazeutische Präparate enthaltend eine Verbindung der Formel (I) gemäss einem der Patentansprüche 1-7 oder 9 oder ein pharmazeutisch annehmbares Salz einer solchen Verbindung, die eine salzbildende Gruppe besitzt und ein β-Lactamantibiotikum.

13. Pharmazeutische Präparate enthaltend die Verbindung gemäss Patentanspruch 8 und ein β-Lactamantibiotikum.

14. Verbindungen der Formel (I) und Salze davon gemäss einem der Patentansprüche 1-7 oder 9 zur Anwendung in einem Verfahren zur therapeutischen Behandlung des menschlichen oder tierischen Körpers.

15. Die Verbindung gemäss Patentanspruch 8 zur Anwendung in einem Verfahren zur therapeutischen Behandlung des menschlichen oder tierischen Körpers.

16. Verbindungen der Formel (I) und Salze davon gemäss einem der Patentansprüche 1-7 oder 9 als β-Lactamase inhibierende Mittel.

17. Die Verbindung gemäss Patentanspruch 8 als β-Lactamase inhibierendes Mittel.

18. Verwendung einer Verbindung der Formel (I) gemäss einem der Patentansprüche 1-7 und 9 und Salzen von solchen Verbindungen, die eine salzbildende Gruppe besitzen, zur Herstellung von pharmazeutischen Präparaten.

19. Verwendung der Verbindung gemäss Patentanspruch 8 zur Herstellung von pharmazeutischen Präparaten.

20. Verfahren zur Herstellung von Verbindungen der Formel (I) und Salzen von solchen Verbindungen, die eine salzbildende Gruppe besitzen, gemäss Patentanspruch 1, dadurch gekennzeichnet, dass man

a) eine Verbindung der Formel

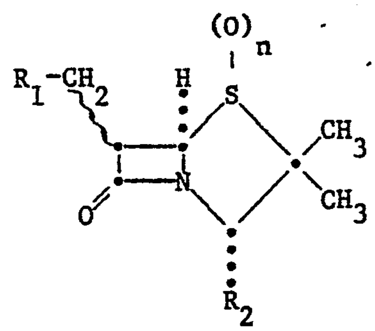

(IIa) ,

worin $R_1$ und $R_2$ die unter Formel (I) genannten Bedeutungen haben und der Index n den Wert 0 oder 1 hat, in 1-Stellung oxidiert, oder

b) in eine Verbindung der Formel

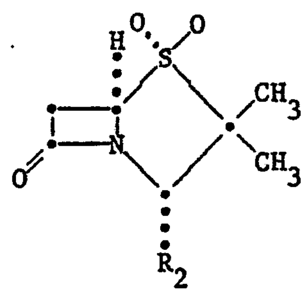

(IIb) ,

worin $R_2$ eine geschützte Carboxylgruppe darstellt, die Gruppe $R_1$-$CH_2$- einführt, oder

c) in einer Verbindung der Formel

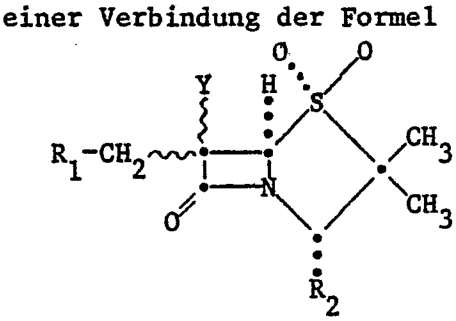

(IIc) ,

worin $R_1$ und $R_2$ die unter Formel (I) genannten Bedeutungen haben und Y eine in ein Wasserstoffatom überführbare Gruppe darstellt, die Gruppe Y in ein Wasserstoffatom überführt, und, wenn erwünscht oder notwendig, in einer erhältlichen Verbindung eine Gruppe $R_1$ in eine andere Gruppe $R_1$ überführt, und/oder eine Gruppe $R_2$ in eine andere

Gruppe $R_2$ überführt, und/oder ein erhältliches Salz in die freie Verbindung oder in ein anderes Salz überführt, und/oder eine erhältliche freie Verbindung mit einer salzbildenden Gruppe in ein Salz überführt, und/oder ein erhältliches Gemisch von isomeren Verbindungen der Formel (I) in die einzelnen Isomeren auftrennt.

21. Verfahren gemäss Anspruch 20, dadurch gekennzeichnet, dass man eine Verbindung der Formel

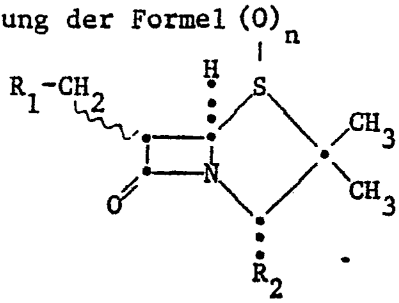

(II) ,

worin $R_1$ und $R_2$ die unter Formel (I) genannten Bedeutungen haben und der Index n den Wert 0 oder 1 hat, in 1-Stellung oxidiert, und, wenn erwünscht oder notwendig, in einer erhältlichen Verbindung eine Gruppe $R_1$ in eine andere Gruppe $R_1$ überführt, und/oder eine Gruppe $R_2$ in eine andere Gruppe $R_2$ überführt, und/oder ein erhältliches Salz in die freie Verbindung oder in ein anderes Salz überführt, und/oder eine erhältliche freie Verbindung mit einer salzbildenden Gruppe in ein Salz überführt, und/oder ein erhältliches Gemisch von isomeren Verbindungen der Formel (I) in die einzelnen Isomeren auftrennt.

22. Die nach den Verfahren gemäss Patentanspruch 20 erhältlichen Verbindungen.

23. Die nach den Verfahren gemäss Patentanspruch 21 erhältlichen Verbindungen.

24. Verbindungen der Formel

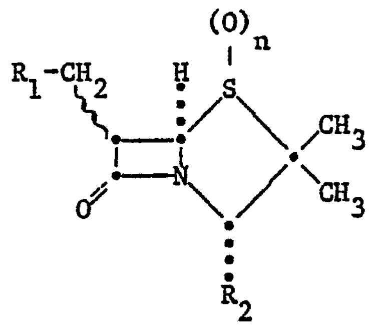

(II) ,

worin $R_1$ Hydroxy oder veräthertes oder veresterte Hydroxy darstellt, $R_2$ Carboxyl oder geschütztes Carboxyl bedeutet und der Index n den Wert 0 oder 1 hat, und Salze von solchen Verbindungen, die eine salzbildende Gruppen besitzen.

25. Verfahren zur Herstellung von Verbindungen der Formel (II) gemäss Patentanspruch 24 und Salzen von sölchen Verbindungen mit salzbildender Gruppe, dadurch gekennzeichnet, dass man

a) in eine Verbindung der Formel

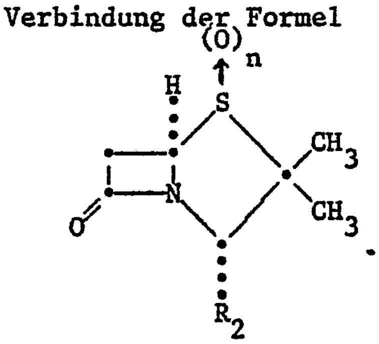

(III) ,

worin der Index n den Wert 0 oder 1 hat und $R_2$ eine veresterte Carboxylgruppe darstellt, die Gruppe $R_1-CH_2-$ einführt, oder

b) in einer Verbindung der Formel

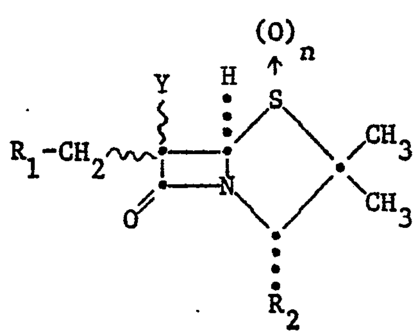

(IV) ,

worin $R_1$, $R_2$ und der Index n die unter Formel (II) genannten Bedeutungen haben und Y eine in ein Wasserstoffatom überführbare Gruppe darstellt, die Gruppe Y in ein Wasserstoffatom überführt, und, wenn erwünscht oder notwendig, in einer erhältlichen Verbindung eine Gruppe $R_1$ in eine andere Gruppe $R_1$ überführt, und/oder eine Gruppe $R_2$ in eine andere Gruppe $R_2$ überführt, und/oder ein erhältliches 1-Sulfid in ein 1-Sulfoxid oder ein erhältliches 1-Sulfoxid in ein Sulfid überführt, und/oder ein erhältliches Salz in die freie Verbindung oder in ein anderes Salz überführt, und/oder eine erhältliche freie Verbindung mit einer salzbildenden Gruppe in ein Salz überführt, und/oder ein erhältliches Gemisch von isomeren Verbindungen der Formel(II) in die einzelnen Isomeren auftrennt.

Patentansprüche (für Oesterreich)

1. Verfahren zur Herstellung von 2,2-Dimethyl-penam-3-carbonsäure-1,1-dioxid-verbindungen der Formel

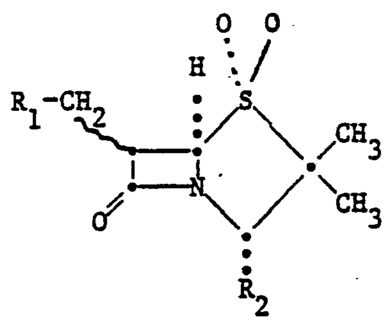

(I) ,

worin $R_1$ Hydroxy oder veräthertes oder verestertes Hydroxy darstellt und $R_2$ Carboxyl oder geschütztes Carboxyl bedeutet, und Salzen von solchen Verbindungen, die eine salzbildende Gruppe besitzen, dadurch gekennzeichnet, dass man

a) eine Verbindung der Formel

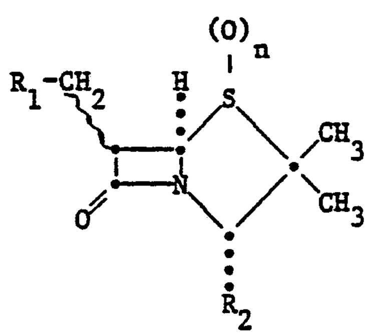

(IIa) ,

worin $R_1$ und $R_2$ die unter Formel (I) genannten Bedeutungen haben und der Index n den Wert 0 oder 1 hat, in 1-Stellung oxidiert, oder

b) in eine Verbindung der Formel

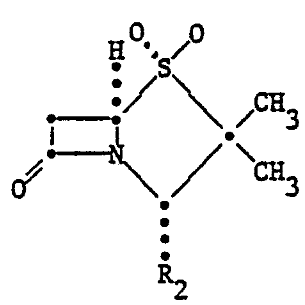

(IIb) ,

worin $R_2$ eine geschützte Carboxylgruppe darstellt, die Gruppe $R_1-CH_2-$ einführt, oder

c) in einer Verbindung der Formel

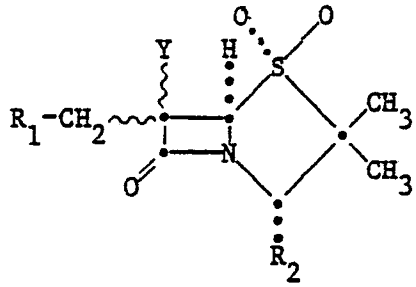

(IIc) ,

worin $R_1$ und $R_2$ die unter Formel (I) genannten Bedeutungen haben und Y eine in ein Wasserstoffatom überführbare Gruppe darstellt, die Gruppe Y in ein Wasserstoffatom überführt, und, wenn erwünscht oder notwendig, in einer erhältlichen Verbindung eine Gruppe $R_1$ in eine andere Gruppe $R_1$ überführt, und/oder eine Gruppe $R_2$ in eine andere Gruppe $R_2$ überführt, und/oder ein erhältliches Salz in die freie Verbindung oder in ein anderes Salz überführt, und/oder eine erhältliche freie Verbindung mit einer salzbildenden Gruppe in ein Salz überführt, und/oder ein erhältliches Gemisch von isomeren Verbindungen der Formel (I) in die einzelnen Isomeren auftrennt.

2. Verfahren gemäss Anspruch 1, dadurch gekennzeichnet, dass man eine Verbindung der Formel

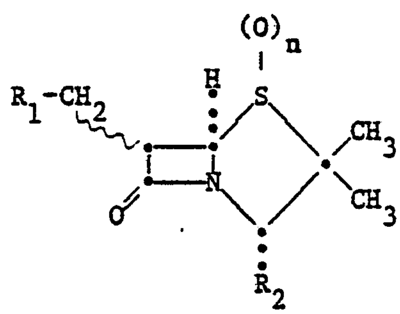

(II) ,

worin $R_1$ und $R_2$ die unter Formel (I) genannten Bedeutungen haben und der Index n den Wert 0 oder 1 hat, in 1-Stellung oxidiert, und,

wenn erwünscht oder notwendig, in einer erhältlichen Verbindung eine Gruppe $R_1$ in eine andere Gruppe $R_1$ überführt, und/oder eine Gruppe $R_2$ in eine andere Gruppe $R_2$ überführt, und/oder ein erhältliches Salz in die freie Verbindung oder in ein anderes Salz überführt, und/oder eine erhältliche freie Verbindung mit einer salzbildenden Gruppe in ein Salz überführt, und/oder ein erhältliches Gemisch von isomeren Verbindungen der Formel (I) in die einzelnen Isomeren auftrennt.

3. Verfahren gemäss Anspruch 1, dadurch gekennzeichnet, dass man Verbindungen der Formel (I), worin $R_1$ Hydroxy, Niederalkyloxy, z.B. Methoxy, Phenylniederalkoxy, z.B. Benzyloxy, Niederalkanoyloxy, z.B. Formyloxy oder Acetoxy, Aminothiazolylcarbonyloxy, z.B. 2-Amino-1,3-thiazol-4-ylcarbonyloxy, Aminothiadiazolylcarbonyloxy, z.B. 5-Amino-1,2,4-thiadiazol-3-yl-carbonyloxy, Niederalkoxycarbonyloxy, Carbamoyloxy, N-Mono- oder N,N-Diniederalkylcarbamoyloxy, z.B. N-Methylcarbamoyloxy oder N,N-Dimethylcarbamoyloxy, Niederalkansulfonyloxy, z.B-Methansulfonyloxy, Arensulfonyloxy, z.B. Benzolsulfonyloxy oder Toluolsulfonyloxy, oder Hydroxysulfonyloxy (oder ein Salz davon, z.B. das Natriumsalz) darstellt und $R_2$ Carboxy oder in physiologisch spaltbarer Form verestertes Carboxy, z.B. Niederalkanoyloxymethoxycarbonyl, z.B. Acetyloxymethyloxycarbonyl oder Pivaloyloxymethoxycarbonyl, Aminoniederalkanoylmethoxycarbonyl, insbesondere α-Amino-niederalkanoyloxymethoxycarbonyl, z.B. Glycyloxymethoxycarbonyl, L-Valyloxymethoxycarbonyl, L-Leucyloxymethoxycarbonyl, Phthalidyloxycarbonyl, z.B. 2-Phthalidyloxycarbonyl, 4-Crotonlactonyl oder gamma-Butyrolacton-4-yl, Indanyloxycarbonyl, z.B. 5-Indanyloxycarbonyl, oder 1-Aethyloxycarbonyloxyäthyloxycarbonyl bedeutet, sowie die pharmazeutisch annehmbaren Salze von solchen Verbindungen, die eine salzbildende Gruppe enthalten, herstellt.

4. Verfahren gemäss Anspruch 1, dadurch gekennzeichnet, dass man

Verbindungen der Formel (I), worin $R_1$ Hydroxy, Methoxy oder Benzyloxy darstellt und $R_2$ Carboxy bedeutet, oder die pharmazeutisch annehmbaren Salze davon, herstellt.

5. Verfahren gemäss Anspruch 1, dadurch gekennzeichnet, dass man Verbindungen der Formel (I), worin $R_1$ Hydroxy ist, herstellt.

6. Verfahren gemäss Anspruch 1, dadurch gekennzeichnet, dass man Verbindungen der Formel (I), deren 6-Stellung die R-Konfiguration besitzt, herstellt.

7. Verfahren gemäss Anspruch 1, dadurch gekennzeichnet, dass man (3R,5R,6R)-2,2-Dimethyl-6-hydroxymethyl-penam-3-carbonsäurediphenylmethylester-1,1-dioxid herstellt.

8. Verfahren gemäss Anspruch 1, dadurch gekennzeichnet, dass man (3R,5R,6R)-2,2-Dimethyl-6-hydroxymethyl-penam-3-carbonsäure-1,1-dioxid-natriumsalz herstellt.

9. Verfahren gemäss Anspruch 1, dadurch gekennzeichnet, dass man (3R,5R,6R)-2,2-Dimethyl-6-hydroxymethyl-penam-3-carbonsäurepivaloyloxymethylester-1,1-dioxid herstellt.

10. Verfahren zur Herstellung von Verbindungen der Formel

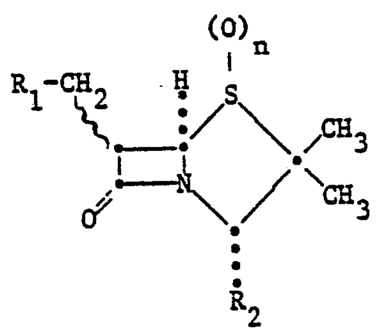

(II) ,

worin $R_1$ Hydroxy oder veräthertes oder verestertes Hydroxy darstellt, $R_2$ Carboxyl oder geschütztes Carboxyl bedeutet und der Index n den Wert 0 oder 1 hat, Salzen von solchen Verbindungen mit salzbildender Gruppe, dadurch gekennzeichnet, dass man

a) in eine Verbindung der Formel

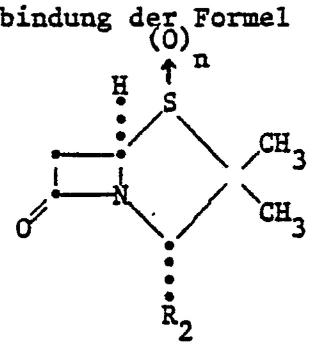

(III) ,

worin der Index n den Wert 0 oder 1 hat und $R_2$ eine veresterte Carboxylgruppe darstellt, die Gruppe $R_1$-$CH_2$- einführt, oder

b) in einer Verbindung der Formel

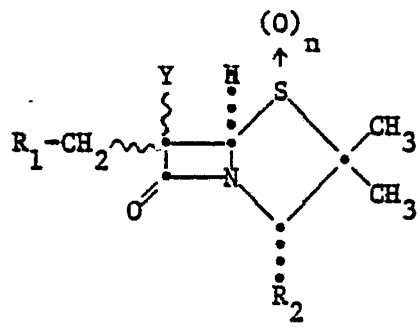

(IV) ,

worin $R_1$, $R_2$ und der Index n die unter Formel (II) genannten Bedeutungen haben und Y eine in ein Wasserstoffatom überführbare Gruppe darstellt, die Gruppe Y in ein Wasserstoffatom überführt, und, wenn erwünscht oder notwendig, in einer erhältlichen Verbindung eine Gruppe $R_1$ in eine andere Gruppe $R_1$ überführt, und/oder eine Gruppe $R_2$ in eine andere Gruppe $R_2$ überführt, und/oder ein erhältliches 1-Sulfid in ein 1-Sulfoxid oder ein erhältliches 1-Sulfoxid in ein 1-Sulfid überführt, und/oder ein erhältliches Salz in die freie Verbindung oder in ein anderes Salz überführt, und/oder eine erhältliche freie Verbindung mit einer salzbildenden Gruppe in ein Salz überführt, und/oder ein erhältliches Gemisch von isomeren Verbindungen der Formel (II) in die einzelnen Isomeren auftrennt.